# EUROPEAN PATENT APPLICATION

(11) **EP 1 676 842 A1**
(43) Date of publication of application: **05.07.2006**
(21) Application number: 04380288.3
(22) Date of filing: 30.12.2004
(51) Int. Cl.: C07D 295/145, C07D 295/096, C07D 207/50, C07D 207/335, C07D 213/74, C07D 231/12, C07D 237/14, A61K 31/495, A61K 31/496, A61P 25/24

(54) **Nitro-substituted phenyl-piperazine compounds, their preparation and use in medicaments**

(71) Applicant: LABORATORIOS DEL DR. ESTEVE, S.A., 08041 Barcelona (ES)
(72) Inventor: Quintana-Ruiz, Jordi Ramon, 08391 Tiana (Barcelona) (ES)
(74) Representative: Carvajal y Urquijo, Isabel

(57) **Abstract**

The present invention relates to nitro-substituted phenyl-piperazine compounds of general formula I, 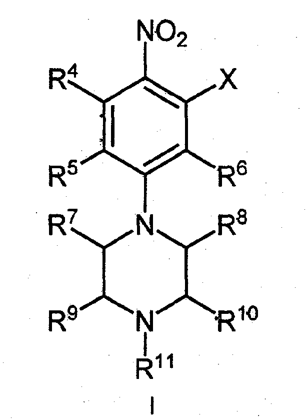 a process for their preparation, medicaments comprising said nitro-substituted phenyl-piperazine compounds as well as the use of said nitre-substituted phenyl-piperazine compounds for the preparation of medicaments, which are particularly suitable for the prophylaxis and/or treatment of disorders or diseases that are at least partially mediated via 5-HT₆ receptors.

## Description

The present invention relates to nitro-substituted phenyl-piperazine compounds of general formula I, 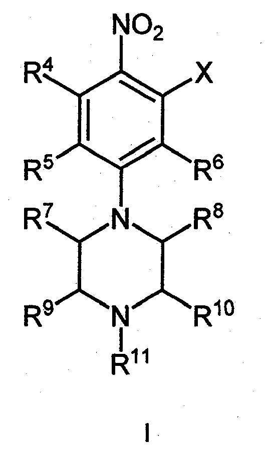 a process for their preparation, medicaments comprising said nitro-substituted phenyl-piperazine compounds as well as the use of said nitro-substituted phenyl-piperazine compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996,66,47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther. , 1995, 274, 173; A.J. Sleight, et al., Behav. Brain Res., 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med. , 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the nitro-substituted phenyl-piperazine compounds of general formula I, Ia and Ib given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one aspect the present invention relates to nitro-substituted phenyl piperazine compounds of general formula I, 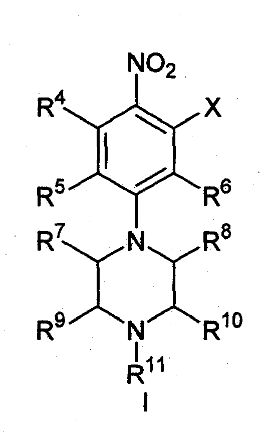 wherein
- X: represents a -NR¹R² moiety or a -OR³ moiety;
- R¹: represents a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may contain 1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I,-CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinofinyl, benzo[b]furanyl, benzo[b]thiophenyl, benzothiadiazolyl and imidazo[2,1-b]thiazolyl, whereby said aryl or heteroaryl radical is bonded via a -(CH₂)-group and may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-C₁₋₅-alkyl,-C(=O)-O-C₁₋₅-alkyl, -O-C(=O)-C₁₋₅-alkyl, F, Br, I, -CN, -CF₃, -OCF₃, -SCF₃,-OH, -SH, -NH2, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅-alkyl,-S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃₋group and/or may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
a -C(=O)-R¹² moiety;
- R²: represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I,-CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂; or
- R¹ and R²: together with the bridging nitrogen a saturated or unsaturated, but not aromatic 3- to 6-membered heterocyclic ring which contains 1, 2 or 3 additional heteroatom(s) independently selected from the group consisting of nitrogen and sulfur as ring member(s) and which may be substituted with 1, 2 or 3 substituent(s) independently selected from the group consisting of C₁₋₅₋alkyl, -O-C₁₋₅-alkyl, -S-C₁₋₅-alkyl, oxo (=O), thioxo(=S), -C(=O)-OH, -C(=O)-C_{1- 5}-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl;
- R³: represents a 5- to 10-membered aryl or heteroaryl radical, which may be unsubstituted or may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅₋alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
- R⁴, R⁵ and R⁶: each represent a hydrogen atom;
- R⁷ and R⁸,: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -M(C₁₋₅-alkyl)₂;
- R⁹ and R¹⁰,: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -N(C₁₋₅-alkyl)₂;
- R¹¹: represents a linear or branched, saturated or unsaturated, unsubstituted C₁₋₁₀ aliphatic radical or a 5- to 10-membered aryl or heteroaryl radical which may be unsubstituted or may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and whereby the heteroaryl radical contains 1, 2 or 3 heteroatom(s) independently selected from the group consisting of nitrogen, oxygen and sulfur as ring member(s);
and
- R¹²: represents an aryl radical which may be substituted with 1, 2, 3, 4 or 5 substituent(s) independently selected from the group consisting of C₁₋₅-alkyl,-O-C₁₋₅-alkyl, -S-C₁₋₅-alkyl, -C(=O)-OH, -C(=O)-C₁₋₅-alkyl,-C(=O)-O-C₁₋₅-alkyl,-O-C(=O)-C₁₋₅-alkyl, F, Br, I, -CN, -CF₃, -OCF₃, -SCF₃, -SH, -NH₂, -NH(C₁₋₅₋alkyl), -N(C₁₋₅-alkyl)₂, -CHO, -CF₂H, -CFH₂, -C(=O)-NH₂, -C(-O)-NH(C₁₋₅₋alkyl), -C(=O)-N(C₁₋₅-alkyl)₂, -S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN,-F₃, -OCF₃, -SCF₃, -OH, -SH, -NH₂, -NH(C₁₋₅-alkyl), -N(C₁₋₅-alkyl)₂, -NO₂,-CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-OH, -C(=O)-C₁₋₅-alkyl, -C(=O)=O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a -(CH₂)_{1, 2 or 3}- group;
optionally in form of one of their stereoisomers, preferably enantiomers or diasteromers, a racemate or in form of a mixture of at least two of their stereoisomers, preferably enantiomers and/or diastereomers, in any mixing ratio, or a physiologically acceptable salt thereof, or a corresponding solvate thereof.

Preferably the compound Benzyl-[5-(4-methyl-piperazin-1-yl)-2-nitro-phenyl]-amine may be excluded in any of the definitions given herein.

Preferred are compounds of general formula I given above, wherein R¹ represents
an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a -(CH₂)_{1, 2 or 3}- group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH3, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a - (CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF3, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -O(=O)-NH-CH₃, - C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
or
a -C(=O)-R¹² moiety;
preferably R¹ represents
an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or
a -C(=O)-R¹² moiety;
more preferably R¹ represents an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, see-butyl, isobutyl, n-pentyl, F, Br and I or
a -C(=O)-R¹² moiety;
and X and R² to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore such nitro-substituted phenyl-piperazine compounds of general formula are preferred, wherein
R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical;
and X, R¹ and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such nitro-substituted phenyl-piperazine compounds of general formula I, wherein
R¹ and R² together with the bridging nitrogen atom form a moiety selected from the group consisting of 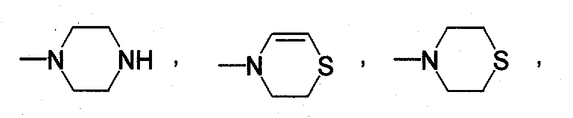 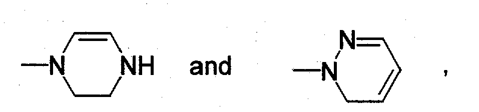 whereby each of these afore mentioned cyclic moieties may be substituted in any position including the -NH-groups with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂₋CH₃, -S(=O)₂-phenyl, phenyl, phenoxy and benzyl;
preferably R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -0-CH₃, -O-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NH-CH₃, -NH-C₂H₅, - NO₂, -CHO, -CF₂H, -CFH₂, -C(=O)-NH₂, phenyl, phenoxy and benzyl;
more preferably R¹ and R² together with the bridging nitrogen atom form an unsubstituted pyridazin-3-one moiety;
and X and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such nitro-substituted phenyl-piperazine compounds of general formula I given above are preferred, wherein

R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1, 2 or 3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C-(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃; -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
more preferably R³ represents an unsubstituted phenyl radical;
and X, R¹, R² and R⁴ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom;
and X, R¹ to R⁶ and R⁹ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom;
and X, R¹ to R⁸ and R¹¹ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein

R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
preferably R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, - C(=O)-OH, -C(=O)-O-CH₃, -C(=O)-O-C₂H₅, F, Cl, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂; -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹¹ represents an alkyl radical selected from the group consisting of methyl, ethyl, n-propyl and n-butyl; or an unsubstituted phenyl or pyridinyl radical;
and X, R¹ to R¹⁰ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R¹² represents an aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O=CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂. -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1, 2 or 3}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH3, -NH-C₂H₅, -NH=CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
preferably R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
more preferably R¹² represents an unsubstituted phenyl radical;
and X and R¹ to R¹¹ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, - C(=O)-CH(CH₃)₂, -C(=O)-C(CH₃)₃, F, Br, I, -CN, -CF₃, -OCF₃, =SCF₃, -OH, -SH, -NH2, -NH-CH₃, -NH-C₂H₅, -NH-CH₂-CH₂₋CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH3)₂, -C(=O)-N(C₂H₅)₂ and -S(=O)₂-CH₃ or a -C(=O)-R¹² moiety;
R² represents a hydrogen atom or an alkyl radical selected from the group consisting of methyl, ethyl, n-propyl, isopropyl, n-butyl, sec-butyl, isobutyl and tert-butyl;
or
R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, - O=C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH; -SH, -NH₂, -NH-CH₃, -NH-C₂H₅, -NO₂, -CHO, -CF₂H, -CFH₂, -C(=O)-NH₂, phenyl, phenoxy and benzyl;
R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C-(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
R⁴, R⁵ and R⁶ each represent a hydrogen atom;
-R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, - C(=O)-O-CH₃, -C(=O)-O=C₂H₅, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and
R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-C-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds of general formula I, wherein
R¹ represents an unsubstituted pyrrolyl radical; an aryl or heteroaryl radical selected from the group consisting of phenyl and pyrrolyl, whereby said phenyl radical is bonded via a-(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or a -C(=O)-R¹² moiety;
R² represents a hydrogen atom or a methyl radical;
or
R¹ and R² together with the bridging nitrogen atom form an unsubstituted pyridazin-3-one moiety;
R³ represents an unsubstituted phenyl radical;
R⁴, R⁵ and R⁶ each represent a hydrogen atom;
R⁷ and R⁸ each represent a hydrogen atom;
R⁹ and R¹⁰ each represent a hydrogen atom;
R¹¹ represents an alkyl radical selected from the group consisting of methyl, ethyl, n-propyl and n-butyl; or an unsubstituted phenyl or pyridinyl radical
and
R¹² represents an unsubstituted phenyl radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2N-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
   optionally in form of a physiologically acceptable salt thereof, or a corresponding solvate thereof.

In yet another aspect the present invention relates to nitro-substituted phenyl-piperazine compounds of general formula I, 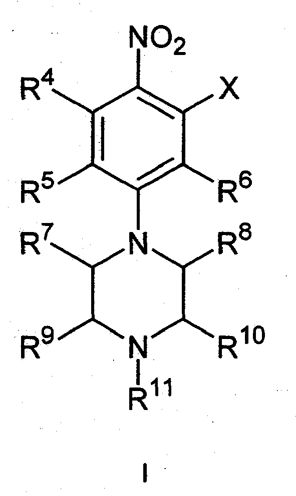 wherein
- X: represents a -NR¹R² moiety or a -OR³ moiety;
- R¹: represents a saturated or unsaturated, optionally at least mono-substituted 3- to 9-membered cycloaliphatic radical, which is bonded via a linear or branched, optionally at least mono-substituted C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s);
an optionally at least mono-substituted heteroaryl radical selected from the group consisting of furyl (furanyl), thienyl (thiophenyl), pyrrolyl, oxazolyl, isoxazolyl, thiazolyl; isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-C₁₋₅-alkyl,-C(=O)-O-C₁₋₅-alkyl, -O-C(=O)-C₁₋₅-alkyl, F, Br, I, -CN, -CF₃, -OCF₃, =SCF₃,-OH, -SH, -NH₂, -NH(C₁₋₅-alkyl), -N(C₁₋₅-alkyl)₂, -NO₂, -CHO, -Cf₂H, -CFH₂,-C(=O)-NH₂, -C(=O)-NH(C₁₋₅-alkyl), -C(=O)-N(C₁₋₅-alkyl)₂, -S(=O)₂-C₁₋₅-alkyl,-S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl;
or
a -C(=O)-R¹² moiety;
- R²: represents a hydrogen atom or a linear or branched, saturated or unsaturated, optionally at least mono-substituted C₁₋₁₀ aliphatic radical;
- R¹ and R²: together with the bridging nitrogen form an optionally at least mono-substituted, saturated or unsaturated, but not aromatic 3- to 9-membered heterocyclic ring which may contain 1, 2 or 3 additional heteroatom(s) independently selected from the group consisting of nitrogen and sulphur as ring member(s);
- R³: represents an optionally at least mono-substituted 5- to 10-membered aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
- R⁴, R⁵ and R⁶: each represent a hydrogen atom;
- R⁷ and R⁸,: identical or different, each represent a hydrogen atom or a linear or branched, saturated or unsaturated, optionally at least mono-substituted C₁₋₁₀ aliphatic radical;
- R⁹ and R¹⁰,: identical or different, each represent a hydrogen atom or a linear or branched, saturated or unsaturated, optionally at least mono-substituted C₁₋₁₀ aliphatic radical;
- R¹¹: represents a linear or branched, saturated or unsaturated, unsubstituted C₁₋₁₀ aliphatic radical or an optionally at least mono-substituted 5- to 10-membered aryl or heteroaryl radical whereby the heteroaryl radical contains 1, 2 or 3 heteroatom(s) independently selected from the group consisting of nitrogen, oxygen and sulphur as ring member(s);
and
- R¹²: represents an aryl radical which may be substituted with 1, 2, 3, 4 or 5 substituent(s) independently selected from the group consisting of C₁₋₅-alkyl,-O-C₁₋₅-alkyl, -S-C₁₋₅-alkyl, -C(=O)-OH, -C(=C)-C₁₋₅-alkyl,-C(=O)-O-C₁₋₅-alkyl,-O-C(=O)-C₁₋₅-alkyl, F, Br, I, -CN, -CF₃, -OCF₃, -SCF₃, -SH, -NH₂, -NH(C₁₋₅₋alkyl), -N(C₁₋₅-alkyl)₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, phenyl, phenoxy and benzyl;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl;
or
an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1, 2 or 3}- group;
with the proviso that the following compound
(1) Benzyl-[5-(4-methyl-piperazin-1-yl)-2-nitro-phenyl]-amine may preferably be excluded;
   optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

If any of the substituents represents or comprises a (hetero)cycloaliphatic radical [(hetero)cycloaliphatic group], said (hetero)cycloaliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -O(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected independently from the group consisting of N, O and S.

Suitable (hetero)cycloaliphatic radicals, which may be unsubstituted or at least mono-substituted, include cyclopropyl, cyclobutyl, cyclopentyl, cyclopentenyl,-cyclohexyl, cyclohexenyl, cycloheptyl, cycloheptenyl, cyclooctyl, -cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl.

If any of the substituents represents or comprises an aryl radical (aryl group), including a phenyl or naphthyl group, said aryl radical may - if not defined otherwise - be unsubstituted or substituted by one or more substituents, preferably unsubstituted or substituted with 1, 2, 3, 4 or 5 substituent(s). Said substituent(s) may pr.eferably be selected independently from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise -be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, -SH, -NH₂, -NH(C₁₋₅-alkyl), -N(C₁₋₅-alkyl)₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If at least two of the substituents together with the bridging nitrogen atom form a saturated or unsaturated heterocyclic ring, said heterocyclic ring may preferably be selected from the group consisting of

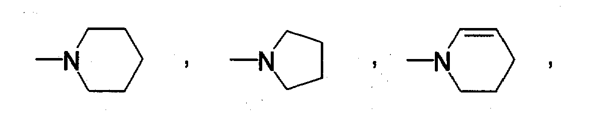

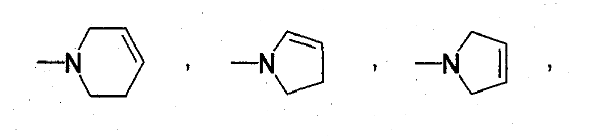

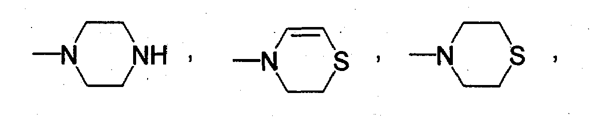

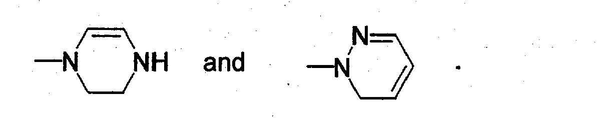

Those skilled in the art understand that the afore mentioned cyclic moieties, which contain an NH group may also be substituted in this position, i.e. the hydrogen atom may be exchanged for another substituent.

Said heterocyclic rings may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO_{2.}

More preferably said substituents may be selected independently from the group consisting of oxo (=O), thioxo (=S), methyl, ethyl, n-propyl, isopropyl, n-butyl, tert-butyl, sec-butyl, isobutyl; n-pentyl, -O-CH₃, -O-C₂H₅; -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, -SCF₃, - OH, -SH, -NH₂, -NH(C₁₋₅-alkyl) and -N(C₁₋₅-alkyl)₂, whereby in each occurence C₁₋₅₋alkyl may be linear or branched. More preferably said substituent(s) may preferably be selected independently from the group consisting of -O-CH₃, -O-C₂H₅, -O-CH₂₋CH2-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

Preferred are compounds of general formula I given above, wherein R¹ represents an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a linear or branched, optionally at least mono-substituted C₁₋₆ alkylene, C₂₋₆ alkenylene or C₂₋₆ alkinylene group and/or may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a-(CH₂)-group and/or may be substituted with 1, 2 or 3 substituent(s) independently selected from the group consisting of C₁₋₅-alkyl, -O-C₁₋₅-alkyl, -S-C₁₋₅-alkyl, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
or
a -C(=O)-R¹² moiety;
preferably R¹ represents an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a -(CH₂)_{1, 2 or 3}- group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a - (CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
or
a -C(=O)-R¹² moiety;
more preferably R¹ represents an unsubstituted pyrrolyl radical; an aryl or heteroaryl radical selected from the group consisting of phenyl and pyrrolyl, whereby said phenyl is bonded via a -(CH₂)-group and said pyrrolyl radical is bonded via a -(CH₂)_{1 or 2}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or a -C(=O)-R¹² moiety
and X and R² to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore such nitro-substituted phenyl-piperazine compounds of general formula I are preferred, wherein R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical;
and X, R¹ and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such nitro-substituted phenyl-piperazine compounds of general formula I, wherein R¹ and R² together with the bridging nitrogen atom form a moiety selected from the group consisting of 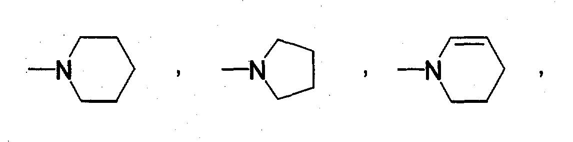 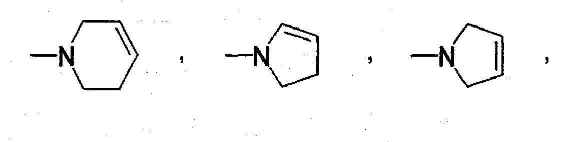 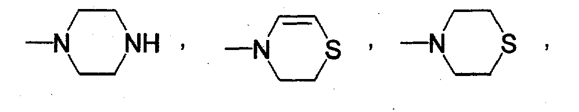 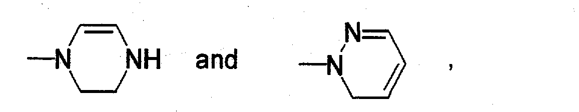 whereby each of these afore mentioned cyclic moieties may be substituted with 1, 2 or 3 substituent(s) independently selected from the group consisting of C₁₋₅-alkyl, -0-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹ and R² together with the bridging nitrogen atom form a moiety selected from the group consisting of 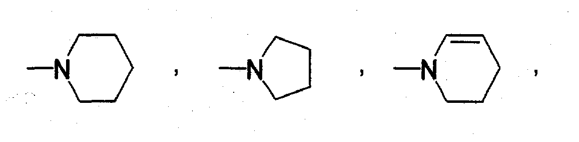 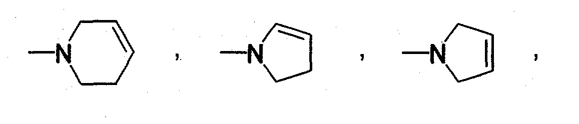 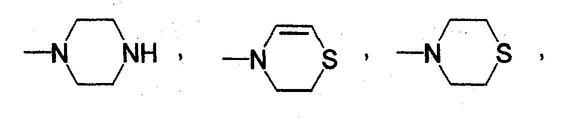 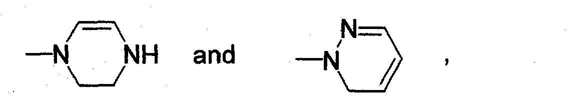 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl;
more preferably R¹ and R² together with the bridging nitrogen atom form a pyridazin-3-one moiety;
and X and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such nitro-substituted phenyl-piperazine compounds of general formula. I given above are preferred, wherein R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a-(CH₂)₁, _{2 or 3}-group and/or may be substituted with 1, 2 or 3 substituent(s) independently selected from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
preferably R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1, 2 or 3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, 1, -CN, -CF₃, -OCF₃, -SCF₃, -OH, =SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, =C(=O)-N(CH₃)₂, -C(=O)-N(C₂H₅)₂ and -S(=O)₂-CH₃,
more preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
and X, R¹, R² and R⁴ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom;
and X, R¹ to R⁶ and R⁹ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom;
and X, R¹ to R⁸ and R¹¹ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R¹¹ represents a linear or branched, unsubstituted C₁₋₁₀ alkyl radical; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; more preferably R¹¹ represents an alkyl radical selected from the group consisting of methyl, ethyl, n-propyl, isopropyl and n-butyl; or an unsubstituted phenyl or pyridinyl radical;
and X, R¹ to R¹⁰ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R¹² represents an aryl radical which may be substituted with 1, 2 or 3 substituent(s) independently selected from the group consisting of whereby said aryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Br, I, -CN, -CF₃, -OCF₃, -SCF₃, - SH, -NH₂, -NH(C₁₋₅-alkyl), -N(C₁₋₅-alkyl)₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl; benzothiadiazolyl and imidazo[2,1-b]thiazolyl whereby said heteroaryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1, 2 or 3}- group and/or may be substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹² represents an aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₁, _{2 or 3}- group and/or may be substituted with 1, 2 or 3 substituent(s) independently selected from the group consisting of methyl, ethyl, n-propyl, isopropyl, n- butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
more preferably R¹² represents a phenyl radical which maybe substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCf₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
and X and R¹ to R¹¹ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
- X: represents a -NR¹R² moiety or a -OR³ moiety;
- R¹: represents an unsubstituted pyrrolyl radical; an aryl or heteroaryl radical selected from the group consisting of phenyl and pyrrolyl, whereby said pheny radical is bonded via a (CH₂)-group and said pyrrolyl radical is bonded via a-(CH₂)_{1 or 2}- group and may be substituted with 1 fluorine atom; or a -C(=O)-R¹² moiety;
- R²: represents a hydrogen atom or a methyl radical;
or
- R¹ and R²: together with the bridging nitrogen atom form a pyridazin-3-one moiety;
- R³: represents an unsubstituted phenyl radical;
- R⁴, R⁵ and R⁶: each represent a hydrogen atom;
- R⁷ and R⁸: each represent a hydrogen atom;
- R⁹ and R¹⁰: each represent a hydrogen atom;
- R¹¹: represents an alkyl radical selected from the group consisting of methyl and n-butyl; or an unsubstituted phenyl or pyridinyl radical and
- R¹²: represents an unsubstituted phenyl radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
   optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Another aspect of the present invention relates to a process for the preparation of a nitro-substituted phenyl-piperazine compound of general formula I wherein X represents -NR¹R², wherein at least one nitrobenzene compound of general formula II, 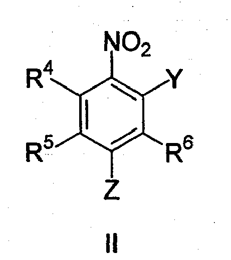 wherein R⁴ to R⁶ have any of the above given meanings, Y represents a chlorine atom, and Z represents a bromine or iodine atom; is reacted with at least one compound of general formula III, 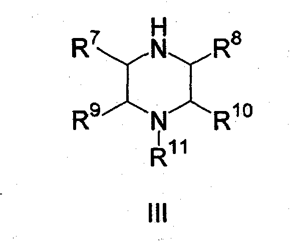 wherein R⁷ to R¹¹ have any of the above given meaningsin a suitable reaction medium, preferably in at least an organic solvent, more preferably in at least an organic solvent selected from the group consisting of tetrahydrofuran, toluene and dioxane, preferably in the presence of at least one catalyst, more preferably in the presence of at least a palladium source, even more preferably in the presence of PdCl₂(dppf) wherein dppf is 1,1-bis(diphenylphosphino)-ferrocene, and/or at least one auxiliary agent, preferably 1,1-bis(diphenylphosphino)-ferrocene, and/or at least one base, preferably sodium *tert*-pentoxide, to yield a compound of general formula IV, 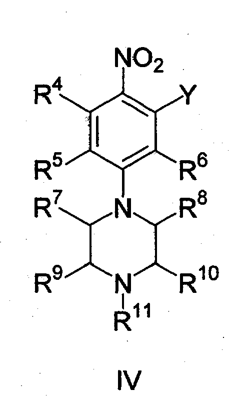 wherein R⁴ to R¹¹ have any of the above given meanings and Y represents a chlorine atom; which is optionally purified and/or isolated, and the compound of general formula IV is reacted with at least one compound of general formula V, 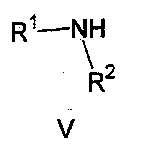 wherein R¹ and R² have any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert-*butyl, and/or at least one base, preferably at least one base selected from the group consisting of K₃PO₄ and sodium *tert*-pentoxide to yield a compound of general formula VI, 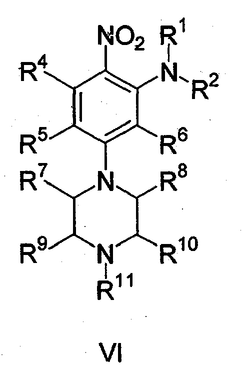 wherein R¹, R² and R⁴ to R¹¹ have any of the above given meanings which is optionally purified and/or isolated.

In another aspect the invention relates to a process for the preparation of a nitro-substituted phenyl-piperazine compound of general formula I wherein X represents -NR¹R², wherein at least one nitrobenzene compound of general formula VII, 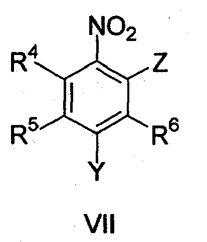 wherein R⁴ to R⁶ have any of the above given meanings, Z represents a bromine or iodine atom, and Y represents a chlorine atom, is reacted with at least one compound of general formula V, 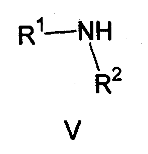 wherein R¹ and R² have any of the above given meaningsin a suitable reaction medium, preferably in at least an organic solvent, more preferably in at least an organic solvent selected from the group consisting of toluene and dioxane, preferably in the presence of at least one catalyst, more preferably in the presence of at least a palladium and/or copper source, even more preferably in the presence of at least a palladium and/or copper source selected from the group consisting of Pd(OAc)₂,
wherein OAc is acetate, Pd₂dba₃, wherein dba is dibenzylidene acetone, and copper(I)iodide, and/or at least one auxiliary agent, preferably at least one auxiliary agent selected from the group consisting of 4,5-bis(diphenylphosphino)-9,9-dimethylxanthene (Xantphos), 1,1-bis(diphenylphosphino-ferrocene and P(*t*Bu)₃,
wherein *t*Bu is tert-Butyl, and/or at least one base, preferably at least one base selected from the group consisting of K₃PO₄, Cs₂CO₃ and trans-1,2-diamino-methylcyclohexane, to yield a compound of general formula VIII, 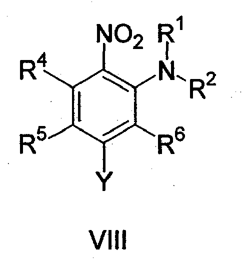 wherein R¹, R² and R⁴ to R⁶ have any of the above given meanings and Y represents a chlorine atom; which is optionally purified and/or isolated, and the compound of general formula VIII is reacted with at least one compound of general formula III, 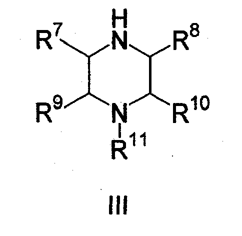 wherein R⁷ to R¹¹ have any of the above given meanings, in a suitable reaction medium, preferably in at least an organic solvent, more preferably in at least an organic solvent selected from the group consisting of toluene, tetrahydrofuran and dimethoxyethane, preferably in the presence of at least one catalyst, more preferably in the presence of at least a palladium source, even more preferably in the presence of at least a palladium source selected from the group consisting of Pd(OAc)₂,
wherein OAc is acetate, and Pd₂dba₃, wherein dba is dibenzylidene acetone, and/or at least one auxiliary agent, preferably (biph)P(*t*Bu)₂, wherein biph is biphenyl and tBu is tert-butyl, and/or at least one base, preferably at least one base selected from the group consisting of K₃PO₄ or sodium *tert*-pentoxide, to yield a compound of general formula VI, 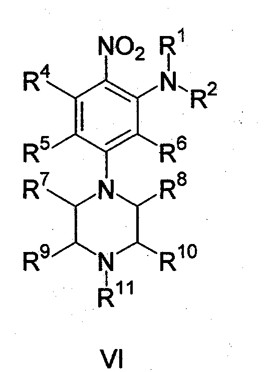 wherein R¹, R² and R⁴ to R¹¹ have any of the above given meanings, which is optionally purified and/or isolated.

Another aspect of the present invention relates to a process for the preparation of a nitro-substituted phenyl-piperazine compound of general formula I wherein X represents -OR³, wherein at least one nitrobenzene compound of general formula II, 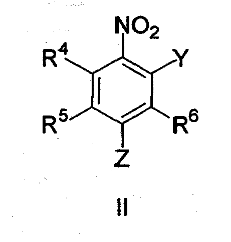 wherein R⁴ to R⁶ have any of the above given meanings, Y represents a chlorine atom, and Z represents a bromine or iodine atom; is reacted with at least one compound of general formula III, 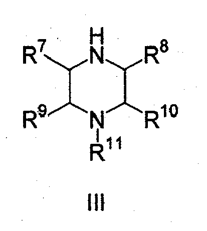 wherein R⁷ to R¹¹ have any of the above given meanings, in a suitable reaction medium, preferably in at least an organic solvent, more preferably in at least an organic solvent selected from the group consisting of tetrahydrofuran or dioxane, preferably in the presence of at least one catalyst, more preferably in the presence of at least a palladium source, even more preferably in the presence of PdCl₂(dppf),
wherein dppf is 1,1-bis(diphenylphosphino)-ferrocene, and/or at least one auxiliary agent, preferably 1,1-bis(diphenylphosphino)-ferrocene, and/or at least one base, preferably sodium *tert*-pentoxide, to yield a compound of general formula IV, 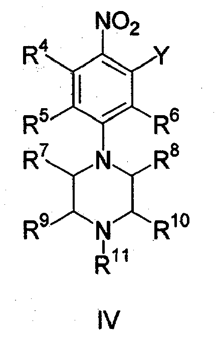 wherein R⁴ to R¹¹ have any of the above given meanings and Y represents a chlorine atom; which is optionally purified and/or isolated, and the compound of general formula IV is reacted with at least one compound of general formula IX, 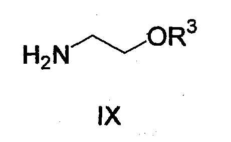 wherein R³ has any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert*-butyl, and/or at least one base, preferably at least one base selected from the group consisting of K₃PO₄ and sodium *tert*-pentoxideto yield a compound of general formula X, 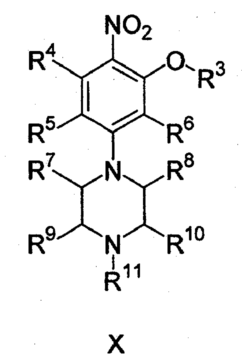 wherein R³ to R¹¹ have any of the above given meanings, which is optionally purified and/or isolated.

Suitable reaction media include organic solvents, such as dialkyl ether, preferably diethyl ether and dimethoxyethane, or a cyclic ether, preferably tetrahydrofuran or dioxane; or a halogenated hydrocarbon, preferably dichloromethane or chloroform; an alcohol, preferably methanol or ethanol; an aprotic solvent, preferably acetonitrile, toluene, pyridine or dimethylformamide, or any other suitable reaction medium. Of course, mixtures of at least two classes of solvents or of at least two solvents of one class may also be used.

All of above mentioned reactions are preferably carried out in an oven-dried vial. The catalyst, the auxiliary agent, the base and the compound of general formula II, IV, VII or VIII are added in each case and the vial is subsequently evacuated and purged with argon. The organic solvent and the compound of general formula III, V or IX are added and the reaction is carried out in a sealed vial at a temperature between 100 °C and 110 °C, preferably at 100 °C in case of tetrahydrofurane or toluene as the organic solvent and at 110 °C in case of dimethoxyethane and dioxane as the organic solvent.

Suitable reaction conditions for carrying out the reaction between compounds of general formula II, IV, VII or VIII and compounds of general formula III, V or IX are described in the references of J.F. Hartwig et al., J. Am. Chem. Soc. 1996, 118, 7217-7218; S.L. Buchwald et al., J. Am. Chem. Soc. 2002, 124, 6043-6048; S. L. Buchwald et al. J. Am. Chem. Soc. 2002, 124, 7241-7424 and S.L. Buchwald et al., J. Am. Chem. Soc. 2002, 124, 11684-11688. The respective part of the description is hereby incorporated by reference and forms part of the present disclosure.

The compounds of general formulas IV, VI, VIII and X given above may be purified and/or isolated according to methods well known to those skilled in the art.

The compounds of general formulas IV, VI, VIII and X may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

Preferably, the compounds of general formula IV, VI, VIII and X may be obtained by filtration of the reaction mixture and subsequent separation of the reaction mixture on a TLC plate. Alternatively, the compounds of general formula I may be isolated by addition of water and methanol to the reaction mixture, evaporating the reaction mixture and purifying the residue by preparative HPLC.

The compounds of general formula II and VII are commercially available or may be prepared according to methods well known in the art, for example, analogous to the methods described in the bibliography of A. McKillop et al., Tetrahedron 1987, 43, 1753. The respective part of the literature description cited above is hereby incorporated by reference and forms part of the disclosure.

The compounds of general formula III, V and IX are commercially available or may be prepared according to methods well known in the art.

During some synthetic reactions described above or while preparing the compounds of general formulas III, V, VI, IX or X the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.
If the nitro-substituted phenyl-piperazine compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents.

The nitro-substituted phenyl-piperazine compounds of general formula I and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

The term "salt" is to be understood as meaning any form of the nitro substituted phenyl-piperazine compounds of general formula I in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the nitro subsituted phenyl-piperazine compounds of general formula I or in each case of corresponding stereoisomers may also be obtained by standard procedures known to those skilled in the art.

The term "solvate" according to this invention is to be understood as meaning any form of the nitro substituted phenyl-piperazine compounds of general formula I in which they have attached to it via non-covalent binding another molecule(most likely a polar solvent) especially including hydrates and alcoholates, e.g. methanolate.

A further aspect of the present invention relates to a medicament comprising at least one nitro-substituted phenyl-piperazine compound of general formula la, 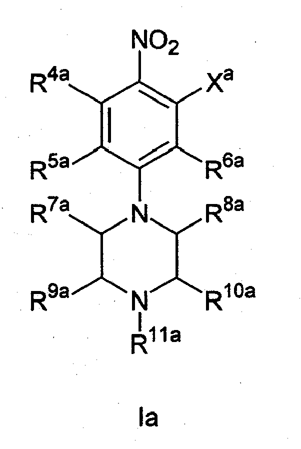 wherein
- X^{a}: represents a -NR^{1a}R^{2a} moiety or a -OR^{3a} moiety;
- R^{1a}: represents a linear or branched, saturated or unsaturated C₁₋₁₀ aliphatic radical which is substituted with 1, 2 or 3 substituent(s) independently selected from the group consisting of F, Cl, Br, I, -CN, -CF₃, -OCF₃, -SCF₃, -OH, -SH, -NH₂,-O-C₁₋₅-alkyl, -S-C₁₋₅-alkyl, -NH-(C₁₋₅-alkyl) and -N(C₁₋₅-alkyl)₂;
a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I,-CN, -CF₃, -OCF₃, -SCF₃, -OH, -SH, -NH₂, -O-C₁₋₅-alkyl, -S-C₁₋₅-alkyl, phenyl,-NH-(C₁₋₅-alkyl) and -N(C₁₋₅-alkyl)₂ and/or which may contain 1, 2 or 3 heteroatom(s) independently selected from the group consisting of nitrogen, oxygen and sulfur as ring member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH,-NH₂, -NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and whereby said heteroaryl radical contains 1, 2 or 3 heteroatom(s) independently selected from the group consisting of nitrogen, oxygen and sulfur as ring member(s);
a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, phenyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s);
a 5- to 10-membered heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl,-O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂-₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O- C₁₋₅-alkyl, -S-C₁₋₅-alkyl, phenyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s) and whereby the heteroaryl radical contains 1, 2 or 3 heteroatom(s) independently selected from the group consisting of nitrogen, oxygen and sulfur as ring member(s);
or a -C(=O)-R^{12a} moiety;
- R^{2a}: represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I,-CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
or
- R^{1a} and R^{2a}: together with the bridging nitrogen form a saturated, unsaturated or aromatic 3- to 6-membered heterocyclic ring which may be substituted with 1, 2 or 3 substituent(s) independently selected from the group consisting of C₁₋₅₋alkyl, -O-C₁₋₅-alkyl, -S-C₁₋₅-alkyl, oxo (=O), thioxo (=S), -C(=O)-OH, -O(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may contain 1, 2 or 3 additional heteroatom(s) independently selected from the group consisting of nitrogen, oxygen and sulfur as ring member(s) and/or which may be condensed with a mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -N02, CHO,-CF₂H, -CFH2, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br,-CN, -CF₃, -OCF₃, -SCF₃, -OH, -SH, -NH₂ and NO₂;
whereby the rings of the ring system are 5- 6- or 7-membered and may contain 1, 2 or 3 heteroatom(s) independently selected from the group consisting of nitrogen, oxygen and sulfur;
- R^{3a}: represents a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃,-SCF₃, -OH, -SH, -NH₂, -NH(C₁₋₅-alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be bonded via a linear or branched C₁₋₆ alkylene, C₂₋₆ alkenylene or C₂₋₆ alkinylene group and which may be unsubstituted or substituted with 1, 2 or 3 substituent(s) independently selected from the group consisting of F, Cl, Br, I, -CN, -CF₃,-OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
- R^{4a}, R^{5a} and R^{6a},: identical or different, each represent a hydrogen atom or a halogen atom
or;
- R^{4a} and R^{5a}: together with the bridging carbon atoms form an unsubstituted 5- or 6-membered heterocyclic ring which contains 1, 2 or 3 heteroatom(s) independently selected from the group consisting of nitrogen, oxygen and sulfur as ring member(s) and which together with the phenyl ring which it is fused with forms a 9- or 10-membered bicyclic aromatic ring system;
- R^{7a} and R^{8a},: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -N(C₁₋₅-alkyl)₂;
- R^{9a} and R^{10a},: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl)-and -N(C₁₋₅-alkyl)₂;
- R^{11a}: represents a hydrogen atom; a linear or branched, saturated or unsaturated C₁₋₁₀ aliphatic radical which may be unsubstituted or substituted with 1, 2 or 3 substituent(s) independently selected from the group consisting of F, Cl, Br, I,-CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCFₛ, -SCF₃, -OH, -SH,-NH₂, -NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched, C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1,2 or 3 heteroatom(s) independently selected from the group consisting of nitrogen, oxygen and sulfur as ring member(s); a -C(=O)-R^{13a} moiety or a -S(=O)₂-R^{14a} moiety;
- R^{12a}: represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃,-OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH,-SH, -NH₂, -NH(C₁₋₅-alkyl), -N(C₁₋₅-alkyl)₂, -NO₂, -CHO, -CF₂H, -CFH₂, -C(=O)-NH2, -C(=O)-NH(C₁₋₅-alkyl), -C(=O)-N(C₁₋₅-alkyl)₂, -S(=O)₂-C₁₋₅-alkyl, -S(=O)₂₋phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
and
- R^{13a} and R^{14a},: identical or different, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅₋alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SOF₃, -OH,-SH, -NH₂, -NH(C₁₋₅-alkyl), -N(C₁₋₅-alkyl)₂, -NO₂, -CHO, -CF₂H, -CFH₂, -C(=O)- NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, phenyl, phenoxy and benzyl and/or which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃,-OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s).
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

Also preferred are medicaments comprising at least one nitro-substituted phenyl piperazine compound of general formula la, wherein
R^{1a} represents an alkyl radical selected from the group consisting of methyl, ethyl, n-propyl, isopropyl, n-butyl, sec-butyl, isobutyl and tert-butyl which is substituted with 1, 2 or 3 substituent(s) independently selected from the group consisting of F, Cl, Br, I, - CN, -CF₃, -OCF₃, -SCF₃, -OH, -SH and -NH₂;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
an aryl radical selected from the group consisting of phenyl and naphthyl which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene group which may contain 1, 2 or 3 oxygen atom(s) as chain member(s) and which may be substituted with 1 phenyl radical;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s);
or a -C(=O)-R^{12a} moiety;
preferably R^{1a} represents an alkyl radical selected from the group consisting of methyl, ethyl, n-propyl and isopropyl, whereby said alkyl radical is substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)=O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and -S(=O)₂₋CH₃ and/or is bonded via an alkylene group selected form the group consisting of - CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of-CH₂-, -CH(CH3)-, -CH₂-CH₂-, -CH₂-CH₂-CH₂- and -CH₂-CH₂-O-;
or a -C(=O)-R^{12a} moiety;
more preferably R^{1a} represents an alkyl radical selected from the group consisting of -CH₂-CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂₋CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂₋CH2-, -CH₂-CH₂-CH₂- and -CH₂-CH₂-O-;
or a -C(=O)-R^{12a} moiety;
and X^{a} and R^{2a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein
R^{2a} represents a hydrogen atom or a linear or branched, unsubstituted C₁₋₁₀ alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical;
and X^{a}, R^{1a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl piperazine compound of general formula la, wherein
R^{1a} and R^{2a} together with the bridging nitrogen atom form a moiety selected from the group consisting of 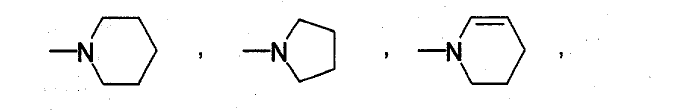 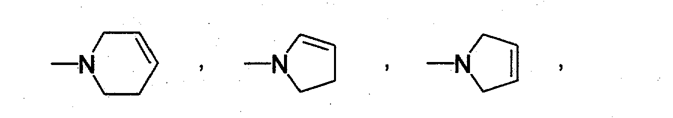 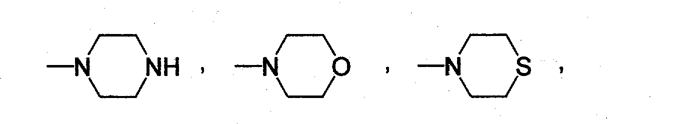 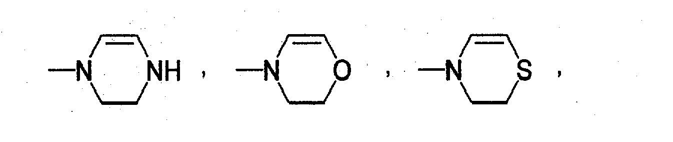 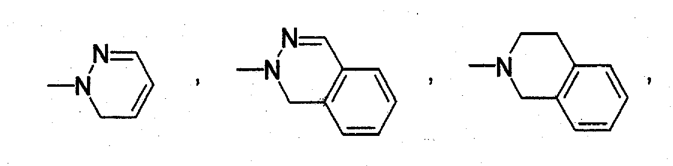 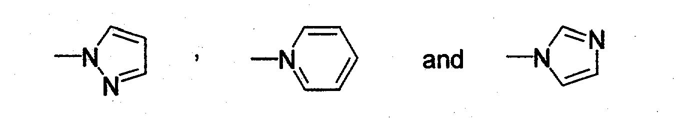 whereby each of these afore mentioned cyclic moieties may be substituted in any position including the -NH-groups with 1, 2 or 3 substituent(s) independently selected from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and NO₂;
preferably R^{1a} and R^{2a} together with the bridging nitrogen atom form a moiety selected from the group consisting of 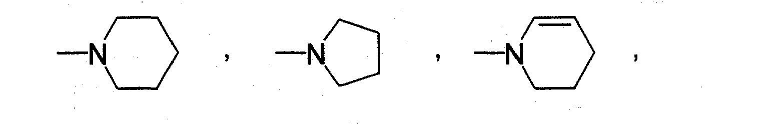 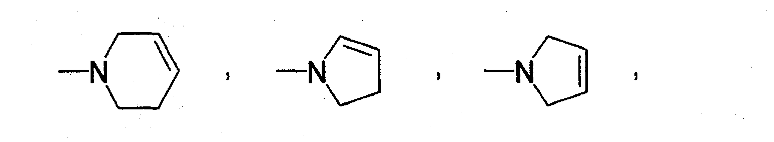 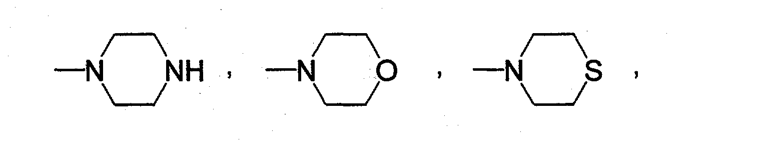 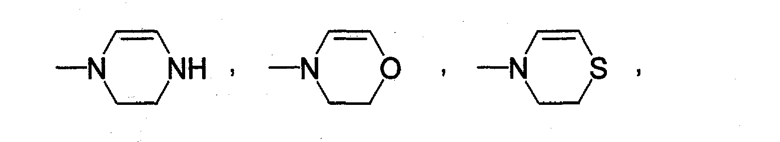 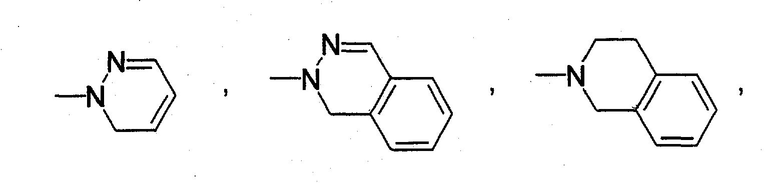 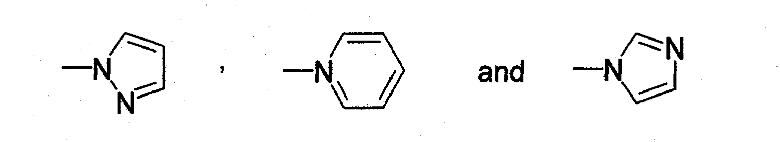 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -0-C(CH₃)₃, -S-CH₃, -S-C₂H₅, -S-CH₂-CH₂-CH₃, -S-CH(CH3)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
more preferably R^{1a} and R^{2a} together with the bridging nitrogen atom form a moiety selected from the group consisting of 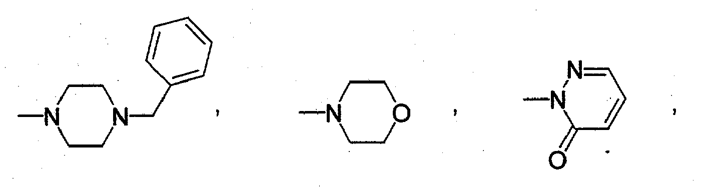 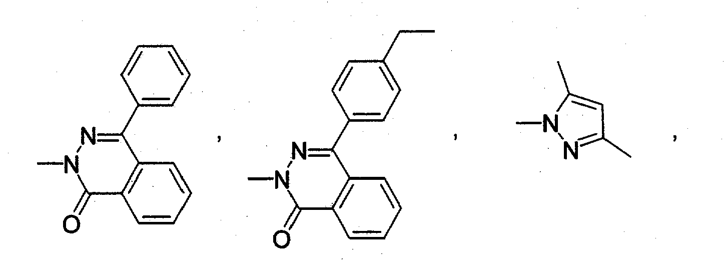 and 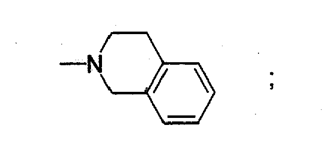 and X^{a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein
R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R^{3a} represents a phenyl radical which may be substituted with 1, 2 or 3 substituent(s) independently selected from the group consisting of methyl, ethyl, n-propyl, isopropyl, n-butyl, tert-butyl, -O-CH₃, -O-C₂H₅, -O-CH₂-CH₂-CH₃, - -C(=O)-OH, -C(=O)-O-CH₃, -C(=O)-O-C₂H₅, -C(=O)-CH₃, -C(=O)-C₂H₅, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂ and -S(=O)₂-CH₃;
more preferably R^{3a} represents an unsubstituted phenyl radical;
and X^{a}, R^{1a}, R^{2a} and R^{4a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such medicaments comprising at least one nitro-substituted phenyl-piperazine compound of general formula Ia are preferred, wherein
R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom;
and X^{a}, R^{1a} to R^{3a} and R^{7a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, whereinR^{4a} and R^{5a} together with the bridging carbon atoms form a moiety selected from the group consisting of 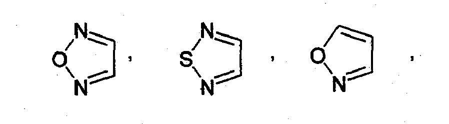 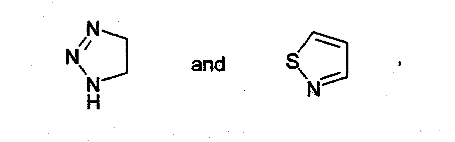 which together with the phenyl ring which it is fused with forms a substituted bicyclic aromatic ring system selected from the group consisting of 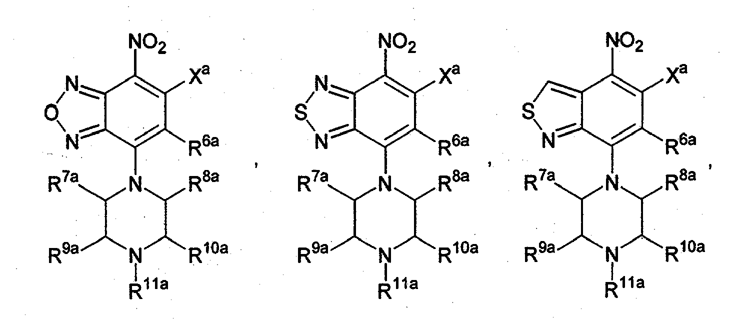 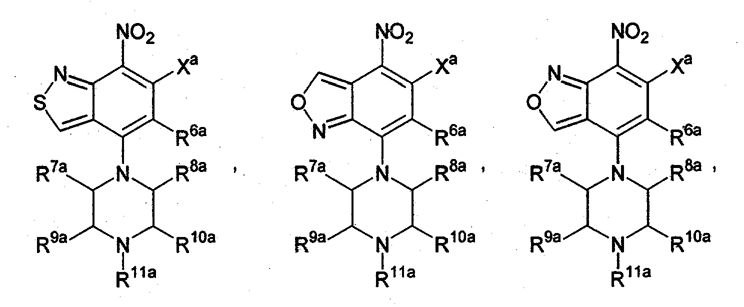 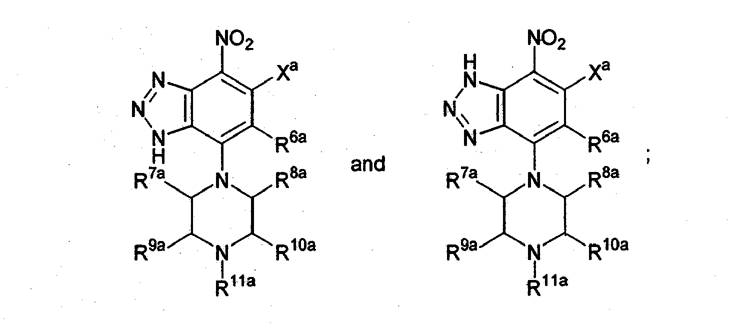 preferably R^{4a} and R^{5a} together with the bridging carbon atoms form the following moiety, 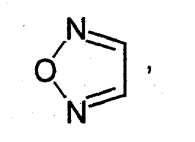 which together with the phenyl ring which it is fused with forms the following substituted bicyclic aromatic ring system 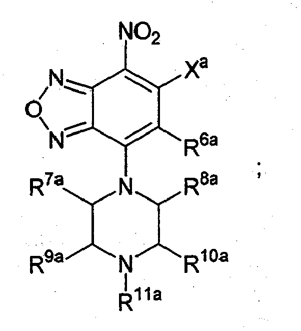 and X^{a}, R^{1a} to R^{3a} and R^{6a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{7a} and R^{8a} each represent a hydrogen atom;
and X^{a}, R^{1a} to R^{6a} and R^{9a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical;
and X^{a}, R^{1a} to R^{8a} and R^{11a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl piperazine compound of general formula la, wherein
R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical - may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅₋alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
a -C(=O)-R^{13a} moiety or a -S(=O)₂-R^{14a} moiety;
preferably R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1,2 or 3}-group;
a -C(=O)-R^{13a} moiety or a -S(=O)₂-R^{14a} moiety;
more preferably R^{11a} represents a hydrogen atom;
an alkyl radical selected from the group consisting of methyl, n-butyl and -CH₂-CH₂₋OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety;
and X^{a}, R^{1a} to R^{10a} and R^{12a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula Ia, wherein
R^{12a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl maybe substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl; -S(=O)₂-phenyl, phenyl, phenoxy and benzyl and/or may be bonded via a -(CH₂)_{1,2 or 3}- group;
preferably R^{12a} represents an alkyl radical selected from the group consisting of methyl, ethyl, n-propyl and isopropyl,
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)- group;
more preferably R^{12a} represents a phenyl or a thiophenyl radical whereby said phenyl or thiophenyl radical may be substituted with 1, 2 or 3 substituent(s) selected from the group consisting of methyl and chlorine;
and X^{a}, R^{1a} to R^{11a}, R^{13a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula Ia, wherein
R^{13a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of-OH, F, Cl, Br and -CN;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, - imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group;
preferably R^{13a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, and thienyl (thiophenyl), whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH3)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃ and/or may be bonded via a -(CH₂)_{1, 2 or 3}-group;
more preferably R^{13a} represents a methyl radical or a phenyl or a thiophenyl radical whereby said phenyl or thiophenyl radical may be substituted with 1, 2 or 3 substituent(s) selected from the group consisting of methyl and chlorine;
and X^{a}, R^{1a} to R^{12a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{14a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
preferably R^{14a} represents an alkyl radical selected from the group consisting of methyl, ethyl, n-propyl and isopropyl,
or a phenyl radical, which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R^{14a} represents a methyl radical or a phenyl radical which may be substituted with 1, 2 or 3 methyl radical(s).

Also preferred are medicaments comprising at least one nitro-substituted phenyl-piperazine compound of general formula Ia, wherein
X^{a} represents a -NR^{1a}R^{2a} moiety or a -OR^{3a} moiety;
R^{1a} represents an alkyl radical selected from the group consisting of -CH₂-CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂₋CH₂-O-;
a heteroaryl radical selected from the group consisting of pyridinyl, furanyl and pyrrolyl, whereby said pyridinyl, furanyl or pyrrolyl radical maybe substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂₋CH₂-, -CH₂-CH₂-CH₂- and -CH₂-CH₂-O-;
or a -C(=O)-R^{12a} moiety;
R^{2a} represents a hydrogen atom or a methyl radical;
or
R^{1a} and R^{2a} together with the bridging nitrogen atom form a moiety selected from the group consisting of 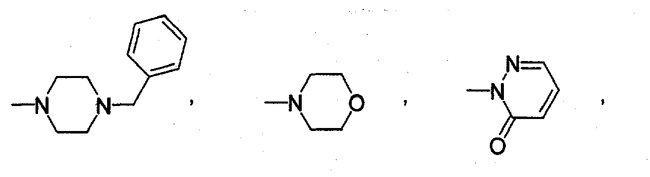 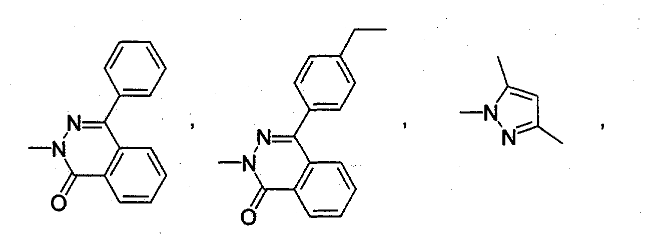 and 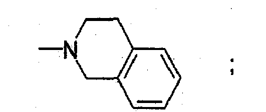 R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 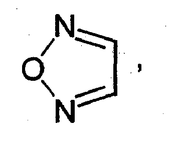 which together with the phenyl ring which it is fused with forms the following substituted bicyclic aromatic ring system 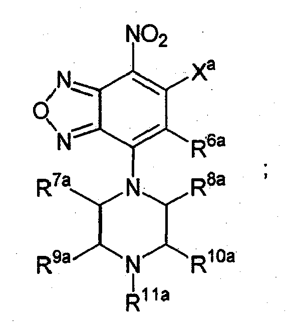 R^{7a} and R^{8a} each represent a hydrogen atom;
R^{9a} and R^{10a}, identical or different, each represent a hydrogen atom or a methyl radical;
R^{11a} represents a hydrogen atom;
an alkyl radical selected from the group consisting of methyl, n-butyl and -CH₂-CH₂₋OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety;
R^{12a} represents a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine
and
R^{14a} represents a methyl radical or a phenyl radical which may be substituted with 1, 2 or 3 methyl radical(s);
optionally in form a physiologically acceptable salt thereof, or a corresponding solvate thereof.

Particularly preferred are medicaments comprising at least one nitro-substituted phenyl-piperazine compound of general formula Ia selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyt)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline;
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methy)-piperazin-1-yl)-2-nitro-pheny]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine;
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chtoro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine and
[60] 1-[4-(3-Benzylamino-4-nitro-phenyl)-piperazin-1-yl]-ethanone,
   optionally in form of a physiologically acceptable salt thereof, or a corresponding solvate thereof.

Yet a further aspect of the present invention relates to a medicament comprising at least one nitro-substituted phenyl-piperazine compound of general formula la, 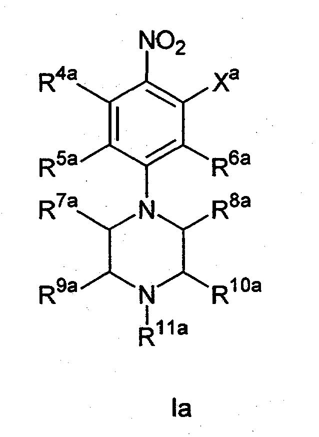 wherein
- X^{a}: represents a -NR^{1a}R^{2a} moiety or a -OR^{3a} moiety;
- R^{1a}: represents a linear or branched, saturated or unsaturated, at least mono-substituted aliphatic radical; a saturated or unsaturated, optionally at least mono-substituted, optionally at least one heteroatom as a ring member containing cycloaliphatic radical, which is bonded via a linear or branched, optionally at least mono-substituted alkylene, alkenylene or alkinylene group; an optionally at least mono-substituted aryl or heteroaryl radical; an aryl radical whereby said aryl radical may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-O-C₁₋₅-alkyl, -O-C(=O)-C₁₋₅-alkyl, F, Cl, Br, I, -ON, -CF₃,-OCF₃, -SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl and said aryl radical is bonded via a linear or branched, optionally at least mono-substituted, optionally at least one heteroatom as a chain member containing alkylene, alkenylene or alkinylene group; an optionally at least mono-substituted heteroaryl radical, which is bonded via a linear or branched, optionally at least mono-substituted, optionally at least one heteroatom as a chain member containing alkylene, alkenylene or alkinylene group; or a -C(=O)-R^{12a} moiety;
- R^{2a}: represents a hydrogen atom or a linear or branched, saturated or unsaturated, optionally at least mono-substituted aliphatic radical;
or
- R^{1a} and R^{2a}: together with the bridging nitrogen form an optionally at least mono-substituted; saturated, unsaturated or aromatic heterocyclic ring which may contain at least one further heteroatom as a ring member and/or which may be condensed with an optionally at least mono-substituted mono- or bicyclic ring system;
- R^{3a}: represents an optionally at least mono-substituted aryl or heteroaryl radical, which may be bonded via a linear or branched, optionally at least mono-substituted alkylene, alkenylene or alkinylene group;
- R^{4a}, R^{5a} and R^{6a},: identical or different, each represent a hydrogen atom or a halogen atom;
or;
- R^{4a} and R^{5a}: together with the bridging carbon atoms form an optionally at least mono-substituted, at least one heteroatom as a ring member containing heterocyclic ring which together with the phenyl ring which it is fused with forms a substituted bicyclic aromatic ring system;
- R^{7a} and R^{8a},: identical or different, each represent a hydrogen atom or a linear or branched, saturated or unsaturated, optionally at least mono-substituted aliphatic radical;
- R^{9a} and R^{10a},: identical or different, each represent a hydrogen atom or a linear or branched, saturated or unsaturated, optionally at least mono-substituted aliphatic radical;
- R^{11a}: represents a hydrogen atom; a linear or branched, saturated or unsaturated, optionally at least mono-substituted, optionally at least one heteroatom as a chain member containing aliphatic radical; an optionally at least mono-substituted aryl or heteroaryl radical, which may be bonded via a linear or branched, optionally at least mono-substituted alkylene, alkenylene or alkinylene group; a -C(=O)-R^{13a} moiety or a -S(=O)₂-R^{14a} moiety;
- R^{12a}: represents a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
and
- R^{13a} and R^{14a},: identical or different, each represent a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

A mono- or bicyclic ring system according to the present invention ― if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅₋alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected from the group consisting of oxo (=O), thioxo (=S), methyl, ethyl, n-propyl, isopropyl, n-butyl, tert-butyl, sec-butyl; isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -O(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents or comprises a (hetero)cycloaliphatic radical [(hetero)cycloaliphatic group], said (hetero)cycloaliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)=N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent{s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected independently from the group consisting of N, O and S.

Suitable (hetero)cycloaliphatic radicals, which may be unsubstituted or at least mono-substituted, include cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl.

If any of the substituents represents or comprises an aryl radical (aryl group), including a phenyl or naphthyl group, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-O(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃; -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If at least two of the substituents together with the bridging nitrogen atom form a saturated or unsaturated heterocyclic ring which may be condensed with an optionally at least mono-substituted mono- or bicyclic ring system, said heterocyclic ring may preferably be selected from the group consisting of

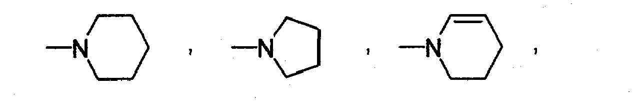

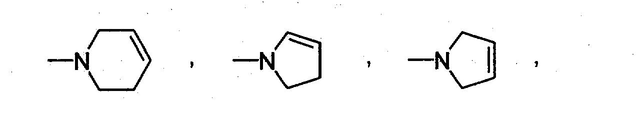

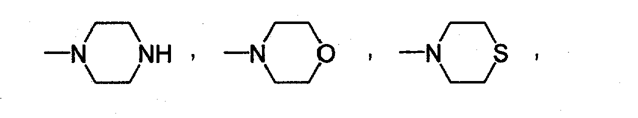

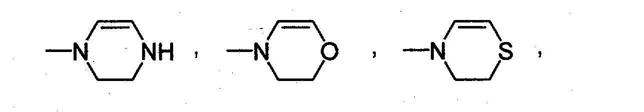

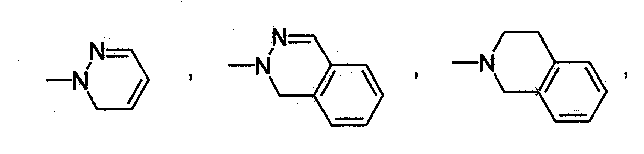

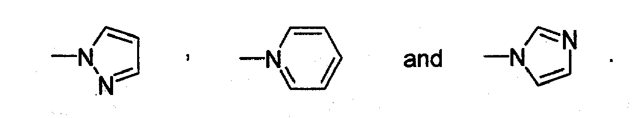

Those skilled in the art understand that the afore mentioned cyclic moieties, which contain an NH group may also be substituted in this position, i.e. the hydrogen atom may be exchanged for another substituent.

Said heterocyclic rings may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -N02, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)2, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, -SCF₃, - OH, -SH, -NH₂, -NH(C₁₋₅-alkyl) and -N(C₁₋₅-alkyl)₂ , whereby in each occurence C₁₋₅₋alkyl may be linear or branched. More preferably said substituent(s) may preferably be selected independently from the group consisting of -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl), -N(C₁₋₅-alkyl)₂ and phenyl, whereby in each occurence C₁₋₅-alkyl may be linear or branched and the phenyl radical is preferably unsubstituted. An alkenylene group comprises at least one carbon-carbon double bond, an alkinylene group comprises at leastone carbon-carbon triple bond.

Suitable alkylene groups include -(CH₂)-, -CH(CH₃)-, -CH(phenyl), -(CH₂)₂-, -(CH₂)₃₋,-(CH₂)₄-,-(CH₂)₅ and -(CH₂)₆-, suitable alkenylene groups include -CH=CH-, -CH₂₋CH=CH- and -CH=CH-CH₂- and suitable alkinylene groups include -C≡C- , -CH₂₋C≡C- and -C≡C-CH₂-.

If the alkylene, alkenylene or alkinylene group contains one or more, preferably 1, 2 or 3, more preferably 1, heteroatom(s) as chain member(s), unless defined otherwise, each of these heteroatom(s) may preferably be selected from the group consisting of N, O and S. Suitable alkylene groups which contain one or more heteroatom(s) include -CH₂-O-CH₂- and -CH₂-CH₂-O.

In another aspect, the invention relates to a medicament comprising at least one nitro-substituted phenyl-piperazine compound of general formula la,
wherein
- X^{a}: represents a -NR^{1a}R^{2a} moiety or a -OR^{3a} moiety;
- R^{1a}: represents a linear or branched, saturated or unsaturated, at least mono-substituted C₁₋₁₀ aliphatic radical; a saturated or unsaturated, optionally at least mono-substituted 3- to 9-membered cycloaliphatic radical, which is bonded via a linear or branched, optionally at least mono-substituted C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical which contains 1, 2 or 3 heteroatom(s) independently selected from the group consisting of nitrogen, oxygen and sulphur as ring member(s); a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl,-S-C₁₋₅-alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl and said aryl radical is bonded via a linear or branched, optionally at least mono-substituted C₁₋₆ alkylene, C₂₋₆ alkenylene or C₂₋₆ alkinylene group which may contain 1, 2 or 3 heteroatom(s) independently selected from the group consisting of nitrogen, oxygen and sulphur as chain member(s); an optionally at least mono-substituted 5- to 10-membered heteroaryl radical, which is bonded via a linear or branched, optionally at least mono-substituted C₁₋₆ alkylene, C₂₋₆ alkenylene or C₂₋₆ alkinylene group which may contain 1, 2 or 3 heteroatom(s) independently selected from the group consisting of nitrogen, oxygen and sulphur as chain member(s) and whereby the heteroaryl radical contains 1, 2 or 3 heteroatom(s) independently selected from the group consisting of nitrogen, oxygen and sulphur as ring member(s); or a -C(=O)-R^{12a} moiety;
- R^{2a}: represents a hydrogen atom or a linear or branched, saturated or unsaturated, optionally at least mono-substituted C₁₋₁₀ aliphatic radical;
or
- R^{1a} and R^{2a}: together with the bridging nitrogen form an optionally at least mono-substituted; saturated, unsaturated or aromatic 3- to 9-membered heterocyclic ring which may contain 1, 2 or 3 additional heteroatom(s) independently selected from the group consisting of nitrogen, oxygen and sulphur as ring member(s) and/or which may be condensed with an optionally at least mono-substituted mono- or bicyclic ring system;
whereby the rings of the ring system are 5- 6- or 7-membered and may contain 1, 2 or 3 heteroatom(s) independently selected from the group consisting of nitrogen, oxygen and sulphur;
- R^{3a}: represents an optionally at least mono-substituted 5- to 10-membered aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
- R^{4a}, R^{5a} and R^{6a},: identical or different, each represent a hydrogen atom or a halogen atom;
or
- R^{4a} and R^{5a}: together with the bridging carbon atoms form an optionally at least mono-substituted 5- or 6- membered heterocyclic ring which contains 1, 2 or 3 heteroatom(s) independently selected from the group consisting of nitrogen, oxygen and sulphur as ring member(s) and which together with the phenyl ring which it is fused with forms a substituted 9- or 10-membered bicyclic aromatic ring system;
- R^{7a} and R^{8a},: identical or different, each represent a hydrogen atom or a linear or branched, saturated or unsaturated, optionally at least mono-substituted C₁₋₁₀ aliphatic radical;
- R^{9a} and R^{10a},: identical or different, each represent a hydrogen atom or a linear or branched, saturated or unsaturated, optionally at least mono-substituted C₁₋₁₀ aliphatic radical;
- R^{11a}: represents a hydrogen atom; a linear or branched, saturated or unsaturated, optionally at least mono-substituted C₁₋₁₀ aliphatic radical which may contain 1, 2 or 3 heteroatom(s) independently selected from the group consisting of nitrogen, oxygen and sulphur as chain member(s); an optionally at least mono-substituted 5- to 10-membered aryl or heteroaryl radical which may be bonded via a linear or branched, optionally at least mono-substituted C₁₋₆alkylene, C₂₋₆ alkenylene or C₂₋₆alkinylene group and whereby the heteroaryl radical contains 1, 2 or 3 heteroatom(s) independently selected from the group consisting of nitrogen, oxygen and sulphur as ring member(s); a -C(=O)-R^{13a} moiety or a -S(=O)₂-R^{14a} moiety;
- R^{12a}: represents a linear or branched, saturated or unsaturated, optionally at least mono-substituted C₁₋₁₀ aliphatic radical; or an optionally at least mono-substituted aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
and
- R^{13a} and R^{14a},: identical or different, each represent a linear or branched, saturated or unsaturated, optionally at least mono-substituted C₁₋₁₀ aliphatic radical; or an optionally at least mono-substituted aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s).

Also preferred are medicaments comprising at least one nitro-substituted phenyl-piperazine compound of general formula la, wherein R^{1a} represents a linear or branched, at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl; an aryl radical selected from the group consisting of phenyl and naphthyl whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via an optionally at least mono-substituted C₁₋₆ alkylene group which may contain 1, 2 or 3 heteroatom(s) independently selected from the group consisting of nitrogen, oxygen and sulphur as chain member(s); an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical is bonded via an optionally at least mono-substituted C₁₋₆ alkylene group which may contain 1, 2 or 3 heteroatom(s) independently selected from the group consisting of nitrogen, oxygen and sulphur as chain member(s); or a -C(=O)-R^{12a} moiety;
preferably R^{1a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of-OH, F, Cl, Br and -CN; an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical maybe substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH2, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; an aryl radical selected from the group consisting of phenyl and naphthyl whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅₋alkyl, -O-C(=O)-C₁₋₅-alkyl, F, CI, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene group which may contain 1, 2 or 3 oxygen atom(s) as chain member{s) and which may be substituted with 1 phenyl radical; a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s); or a -C(=O)-R^{12a} moiety;
more preferably R^{1a} represents an alkyl radical selected from the group consisting of methyl, ethyl, n-propyl and isopropyl, whereby said alkyl radical may be substituted with 1, 2 or 3 substituent(s) independently selected from the group consisting of -OH, F, Cl, Br and -CN; an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and -S(=O)₂₋CH₃ and/or is bonded via an alkylene group selected form the group consisting of - CH₂-, -CH(CH₃)-, -CH(Phenyl)-, -CH₂-CH₂-, -CH₂-CH₂-CH₂- and -CH₂-CH₂-O-; a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-MC₂H₅)₂ and -S(=O)₂-CH₃ and/or is bonded via an alkylene group selected form the group consisting of -CH₂-, -CH(CH₃)-, -CH₂-CH₂-, -CH₂-CH₂-CH₂- and -CH₂-CH₂-O-; or a - C(=O)-R^{12a} moiety;
and X^{a} and R^{2a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein R^{2a} represents a hydrogen atom or a linear or branched, unsubstituted C₁₋₁₀ alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical;
and X^{a}, R^{1a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein R^{1a} and R^{2a} together with the bridging nitrogen atom form a moiety selected from the group consisting of 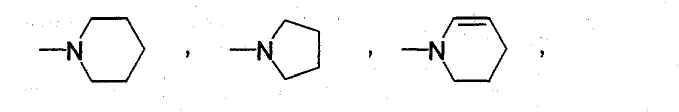 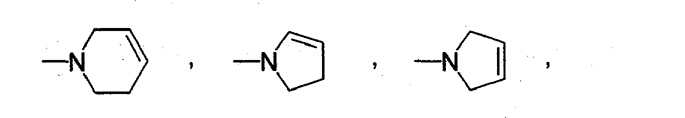 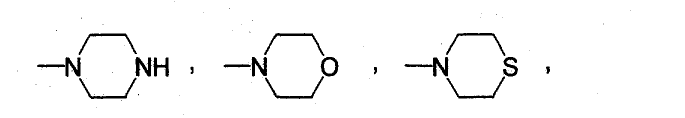 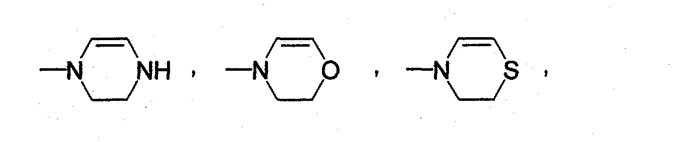 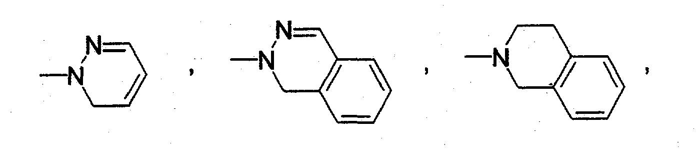 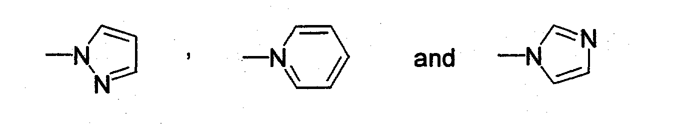 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and NO₂;
preferably R^{1a} and R^{2a} together with the bridging nitrogen atom form a moiety selected from the group consisting of 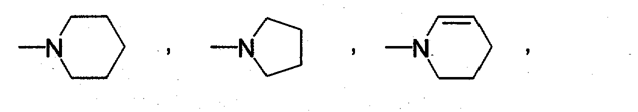 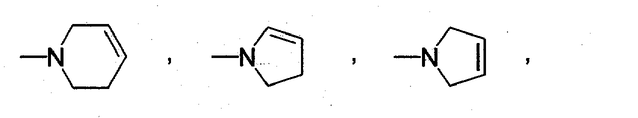 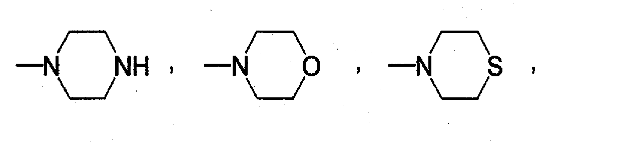 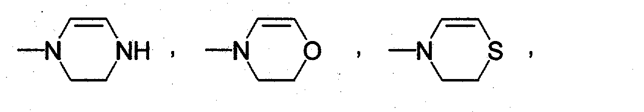 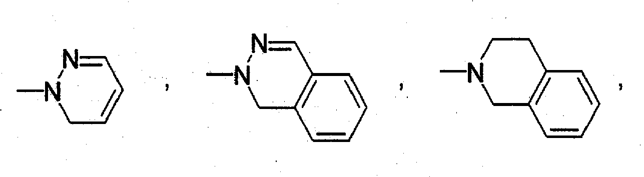 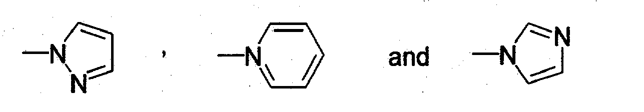 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C-(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
more preferably R^{1a} and R^{2a} together with the bridging nitrogen atom form a moiety selected from the group consisting of 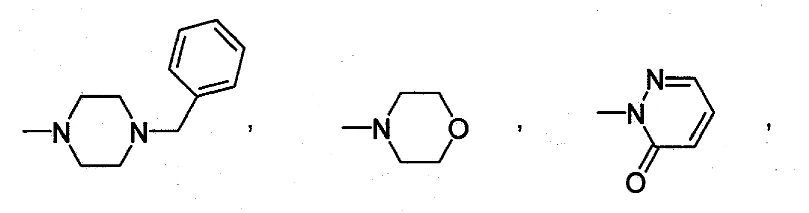 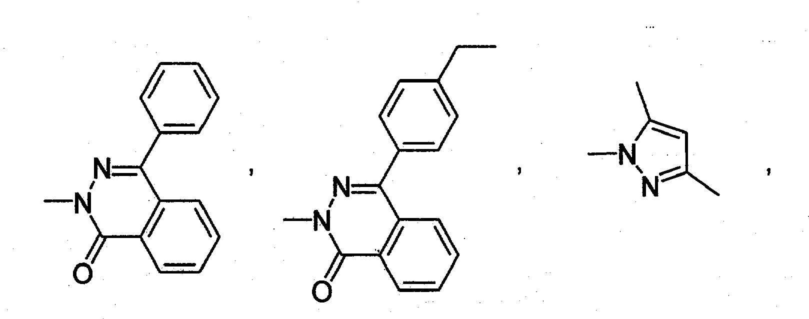 and 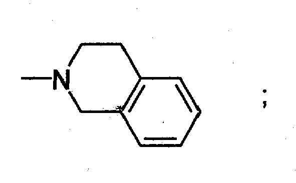 and X^{a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula Ia, wherein R^{3a} represents an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via an optionally at least mono-substituted C₁₋₆ alkylene group;
preferably R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be substituted with 1, 2, 3, 4 or 5 substituent(s) independently selected from the group consisting of C₁₋₅-alkyl, -O-C₁₋₅₋alkyl, -S-C₁₋₅-alkyl, -C(=O)-OH, -C(=O)-O-C₁₋₅-alkyl, -O-C(=O)-C₁₋₅-alkyl, F, Cl, Br, I,-CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl and said aryl or heteroaryl radical may be bonded via a linear or branched C₁₋₆ alkylene group;
more preferably R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂₋CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C-(=O)-CH₃, -O(=O)-C₂H₅, -C(=O)-CH₂-CH₂-CH₃, -C(=O)-CH(CH₃)₂, -C(=O)-C(CH₃)₃. F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and X^{a}, R^{1a}, R^{2a} and R^{4a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such medicaments comprising at least one nitro-substituted phenyl-piperazine compound of general formula la are preferred, wherein R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom;
and X^{a}, R^{1a} to R^{3a} and R^{7a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein R^{4a} and R^{5a} together with the bridging carbon atoms form a moiety selected from the group consisting of 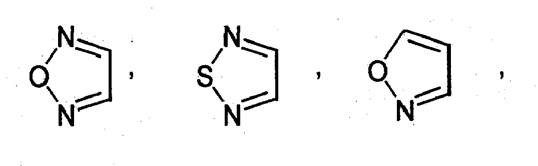 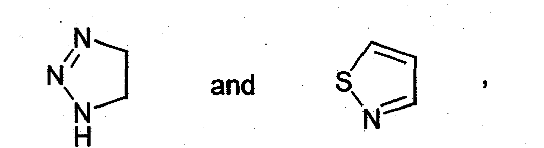 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂ and - NO₂; which together with the phenyl ring which it is fused with forms a substituted bicyclic aromatic ring system selected from the group consisting of 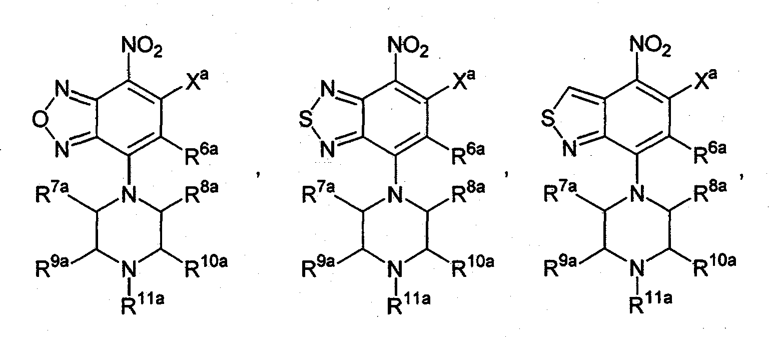 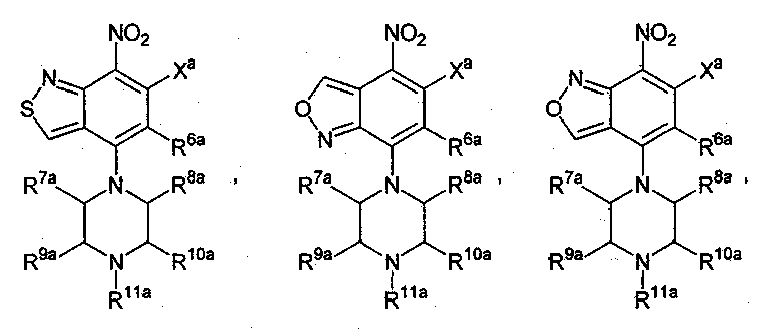 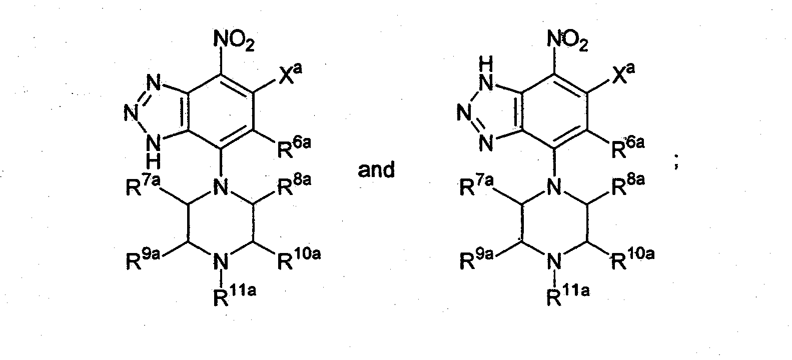 preferably R^{4a} and R^{5a} together with the bridging carbon atoms form the following moiety, 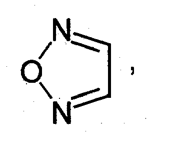 which together with the phenyl ring which it is fused with forms the following substituted bicyclic aromatic ring system 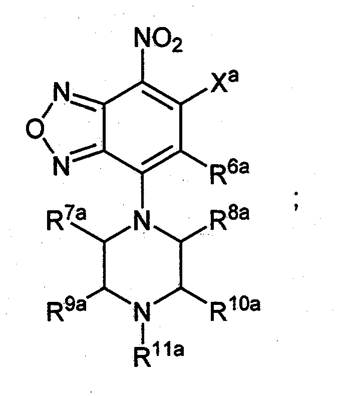 and X^{a}, R^{1a} to R^{3a} and R^{6a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group-consisting of methyl, ethyl, n-propyl, isopropyl, n-butyl, sec-butyl, isobutyl and tert-butyl;
more preferably R^{7a} and R^{8a} each represent a hydrogen atom;
and X^{a}, R^{1a} to R^{6a} and R^{9a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical;
and X^{a}, R^{1a} to R^{8a} and R^{11a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl piperazine compound of general formula la, wherein R^{11a} represents a hydrogen atom; a linear or branched, optionally at least mono-substituted C₁₋₁₀ alkyl radical; an aryl or heteroaryl radical selected from the group consisting of phenyl and pyridinyl whereby said aryl or heteroaryl radical is substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, -CN, -OCF₃, -SCF₃, -OH, -SH, -NH₂, -NH(C₁₋₅-alkyl), -N(C₁₋₅-alkyl)₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and/or may be bonded via a C₁₋₆₋alkylene group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; a - C(=O)-R^{13a} moiety or a =S(=O)₂-R^{14a} moiety;
preferably R^{11a} represents a hydrogen atom; an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN; an aryl or heteroaryl radical selected from the group consisting of phenyl and pyridinyl whereby said aryl or heteroaryl radical is substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, -CN, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃ and/or may be bonded via a - (CH₂)_{1, 2 or 3}-group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -N02, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group; a - C(=O)-R^{13a} moiety or a -S(=O)₂-R^{14a} moiety;
and X^{a}, R^{1a} to R^{10a} and R^{12a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{12a} represents a linear or branched, optionally at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyi, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be bonded via a -(CH₂)_{1,2} or 3⁻ group;
preferably R^{12a} represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
more preferably R^{12a} represents an alkyl radical selected from the group consisting of methyl, ethyl, n-propyl and isopropyl, or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂) - group;
and X^{a}, R^{1a} to R^{11a}, R^{13a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula Ia, wherein R^{13a} represents a linear or branched, optionally at least mono-substituted C₁₋₁₀ alkyl radical; an aryl or heteroaryl radical selected from the group consisting of phenyl and pyridinyl whereby said aryl or heteroaryl radical is substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, -CN, -OCF₃, -SCF₃, - OH, -SH, -NH₂, -NH(C₁₋₅-alkyl), -N(C₁₋₅-alkyl)₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; a -C(=O)-R^{13a} moiety or a -S(=O)₂-R^{14a} moiety;
preferably R^{13a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN; an aryl or heteroaryl radical selected from the group consisting of phenyl and pyridinyl whereby said aryl or heteroaryl radical is substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, -CN, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH2-CH₃, -C(=O)-O-CH(CH₃)₂, -C(=O)-O-C(CH₃)₃, -C(=O)-CH₃, -C(=O)-C₂H₅, - C(=O)-CH₂-CH₂₋CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃ and/or may be bonded via a -(CH₂)_{1, 2} or ₃-group; a -C(=O)-R^{13a} moiety or a -S(=O)₂-R^{14a} moiety;
and X^{a}, R^{1a} to R^{12a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula Ia, wherein R^{14a} represents a linear or branched, optionally at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be bonded via a -(CH₂)_{1,2} or ₃- group;
preferably R^{14a} represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
more preferably R^{14a} represents an alkyl radical selected from the group consisting of methyl, ethyl, n-propyl and isopropyl, or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-O(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)- group;
and X^{a} and R^{1a} to R^{13a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula Ia, wherein
- X^{a}: represents a -NR^{1a}R^{2a} moiety or a -OR^{3a} moiety;
- R^{1a}: represents an alkyl radical selected from the group consisting of -CH₂-CH₂₋OH and -CH₂-CH₂-CH₂-OH; an unsubstituted phenyl or pyrrolyl radical; a phenyl radical which may be substituted with 1, 2 or 3 substituent(s) independently selected from the group consisting of methyl, ethyl, tert-butyl, methoxy, F and Cl and said is bonded via an alkylene group selected form the group consisting of -CH₂-, -CH(CH₃)-, -CH(Phenyl)-, -CH₂-CH₂-, -CH₂-CH₂₋CH₂- and -CH₂-CH₂-O-; a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -OH₂-CH₂₋CH₂- and -CH₂-CH2-O-; or a -C(=O)-R^{12a}, moiety;
- R^{2a}: represents a hydrogen atom or a methyl radical;
or 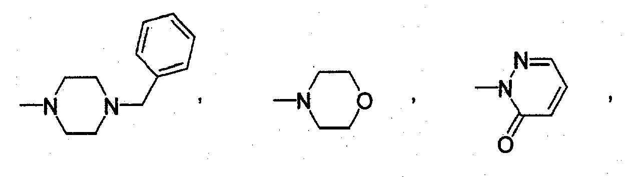 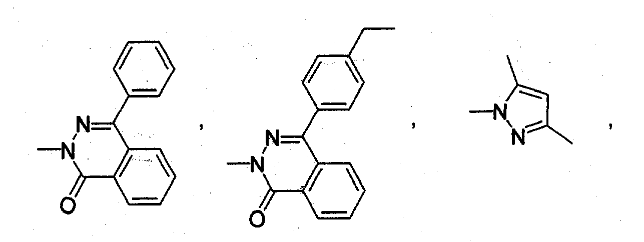 and 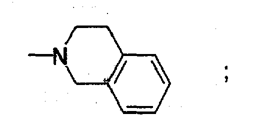 R^{1a} and R^{2a} together with the bridging nitrogen atom form a moiety selected from the group consisting of
R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 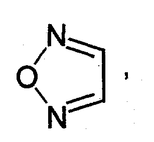 which together with the phenyl ring which it is fused with forms the following substituted bicyclic aromatic ring system 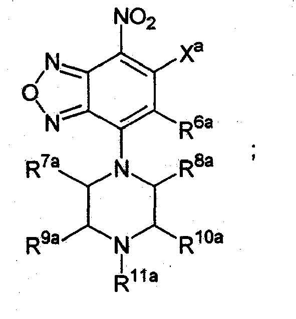 R^{7a} and R^{8a} each represent a hydrogen atom;
R^{9a} and R^{10a}, identical or different, each represent a hydrogen atom or a methyl radical;
R^{11a} represents a hydrogen atom; an alkyl radical selected from the group consisting of methyl, n-butyl and -CH₂-CH₂-OH; an unsubstituted phenyl or pyridinyl radical whereby said phenyl or pyridinyl radical may be bonded via a -(CH₂)- group; a -C(=O)-R¹² moiety or a -S(=O)₂-R^{13a} moiety;
R^{12a} represents a methyl radical or a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine;
and
R^{14a} represents a methyl radical or a phenyl radical which may be substituted with 1, 2 or 3 methyl radical(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are medicaments comprising at least one nitre-substituted phenyl-piperazine compound of general formula la selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1 -yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-pipierazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyi]-[5-(4-methyl-piperazin-1-yl)-2-nitrophenyl]-amine,
[51 [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine and
[60] 1-[4-(3-Benzylamino-4-nitro-phenyl)-piperazin-1-yl]-ethanone,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Said medicament is particularly suitable for 5-HT₆-receptor regulation and therefore for the prophylaxis and/or treatment of a disorder or a disease that is least partially mediated via 5-HT₆-receptors.

Preferably said medicament is suitable for the prophylaxis and/or treatment of a disorder or disease related to food intake, preferably for the regulation of appetite; for the maintenance, increase or reduction of body weight; for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal or for the prophylaxis and/or treatment of nicotine addiction and/or withdrawal.

More preferably said medicament is suitable for the prophylaxis and/or treatment of a disorder or disease related to food intake, preferably for the regulation of appetite; for the maintenance, increase or reduction of body weight; for the prophylaxis and/or treatment of obesity, bulimia, anorexia, cachexia or type II diabetes (non insulin dependent diabetes mellitus), preferably type II diabetes that is caused by obesity.

Furthermore, more preferably said medicament is suitable for improvement of cognition (cognitive enhancement) or cognitive memory (cognitive memory enhancement).

Furthermore, more preferably said medicament is suitable for the prophylaxis and/or treatment of drug addiction and/or withdrawal; for the prophylaxis and/or treatment of alcohol addiction and/or withdrawal or for the prophylaxis and/or treatment of nicotine addiction and/or withdrawal.

Most preferably, said medicament is suitable for the prophylaxis and/or treatment of obesity and/or disorders or diseases related thereto.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-substituted phenyl-piperazine compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more nitro-substituted phenyl-piperazine compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000 mg, preferably 1 to 1500 mg, more preferably 1 to 1000 milligrams of active substance to be administered during one or several intakes per day.

The use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity is preferred.

The use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder) is preferred.

Also preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

Also preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

More preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

More preferred is the use of at least one nitro-substituted phenyl-piperazine compound of general formula I or Ia given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

More preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

Most preferred is the use of at least one nitro-substituted phenyl-piperazine compound of general formula I or la as defined above, optionally in form of one of its, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of obesity and/or disorders or diseases related thereto.

In the following methods for determining the pharmacological activity of the nitro-substituted phenyl-piperazine compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 pg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted nitro-substituted phenyl-piperazine compounds according to the present invention in fasted rats is then determined as follows:

The rats were fasted for 23 hours in their single homecages. After this period, the rats are orally or intraperitoneally dosed with a composition comprising a nitro-substituted phenyl-piperazine compound or a corresponding composition (vehicle) without said nitro-substituted phenyl-piperazine compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples

### Abbreviations

- aq.: aqueous
- biph: biphenyl
- br: broad
- d: doublett
- dba: dibenzylidene acetone
- DCM: dichlormethane
- DME: 1,2-dimethoxyethan
- dppf: 1,1-bis(diphenylphosphino-ferrocene
- EA: ethyl acetate
- eq.: equivalent
- h: hours
- HPLC: high performance liquid chromatography
- m: multiplett
- MeOH: methanol
- OAc: acetate
- O*t*Pent: *tert*-pentoxide
- PE: petroleum ether
- prep.: preparative
- s: singulett
- t: triplett
- *t*Bu: tert-butyl
- THF: tetrahydrofuran
- TLC: thin layer chromatography
- xantphos: 4,5-bis(diphenylphosphino)-9,9-dimethylxanthene

### Preparation of example compounds 1, 2, 5, 7, 8, 9 and 10

### Step 1.

The starting material 4-bromo-2-chloro-1-nitro-benzene was obtained by oxidation from 4-bromo-2-chloro-aniline according to the method described in the reference by A. McKillop et al., Tetrahedron 1987, 43,1753.

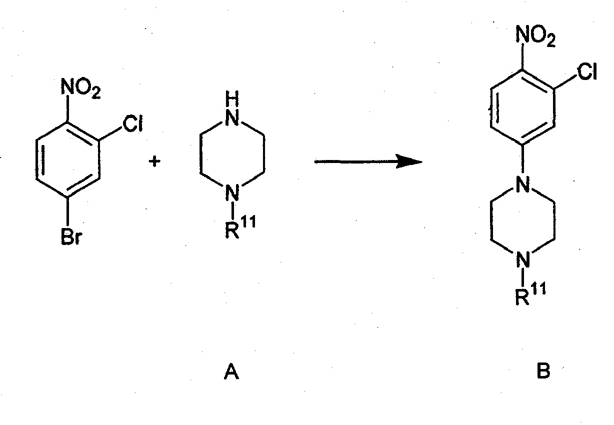

An oven-dried vial was charged subsequently with 0.05 eq. PdCl₂(dppf), 0.15 eq. dppf, 1.0 eq. 4-bromo-2-chloro-1-nitro-benzene and 1.25 eq. sodium *tert*-pentoxide. The vial was evacuated and purged with argon, and THF and piperazine of general formula A (1.1 eq.) were added. THF was added in a quantity to obtain a 0.5 M solution of 4-bromo-2-chloro-1-nitro-benzene. The vial was sealed and heated to 100°C for 3 h.
The reaction mixture was taken up in half-saturated brine and extracted three times with ethyl acetate. The volume of the combined organic phases was reduced in vacuo and extracted with 5% hydrochloric acid in water. The acidic aqueous phase was concentrated and the product was isolated by preparative HPLC. In order to remove any ammonium formate derivative formed during preparative HPLC on RP18, the resulting product was taken up in chloroform, extracted twice with saturated aqueous NaHCO₃, the combined aqueous phases were re-extracted with chloroform, and the combined organic phases were dried over MgSO₄ and concentrated in vacuo to obtain the desired product.

Said process was carried out according to the methode described in the reference of J. F. Hartwig et al., J. Am. Chem. Soc. 1996, 118, 7217-7218. The respective part of the description is hereby incorporated by reference and forms part of the present disclosure.

### Step 2.

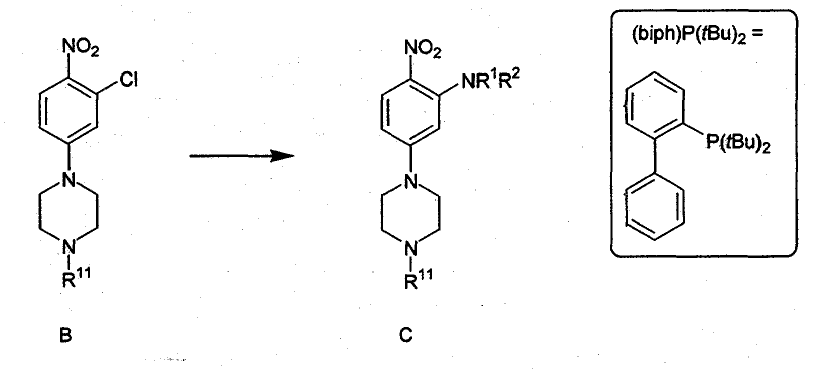

An oven-dried vial was subsequently charged with 0.1 eq. of a palladium source [Pd], 0.2 eq. (biph)P(*t*Bu)₂, 1.0 eq. arylhalide BI and 1.4 eq. base. The vial was evacuated and purged with argon, and the respective amine HNR¹R² (1.3 eq.) was added. The solvent was added to obtain a concentration of 1.0 M of the arylhalide B. The vial was sealed and heated to either 100°C in case of toluene as the solvent or 110°C in case of DME as the solvent in each case for 22 h. The mixture was worked up differently.

### Work-up 1:

The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA) mixtures.

### Work-up 2:

The reaction mixture was transferred into a flask using H₂O and MeOH and the total volume was reduced to less than 10 mL. Purification was achieved by prep. HPLC. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

The different reaction conditions and work-up procedures are listed in table 1. Said process was carried out according to the methode described in the reference of S.L. Buchwald et al. J. Am. Chem. Soc. 2002, 124, 6043-6048. The respective part of the description is hereby incorporated by reference and forms part of the present disclosure.

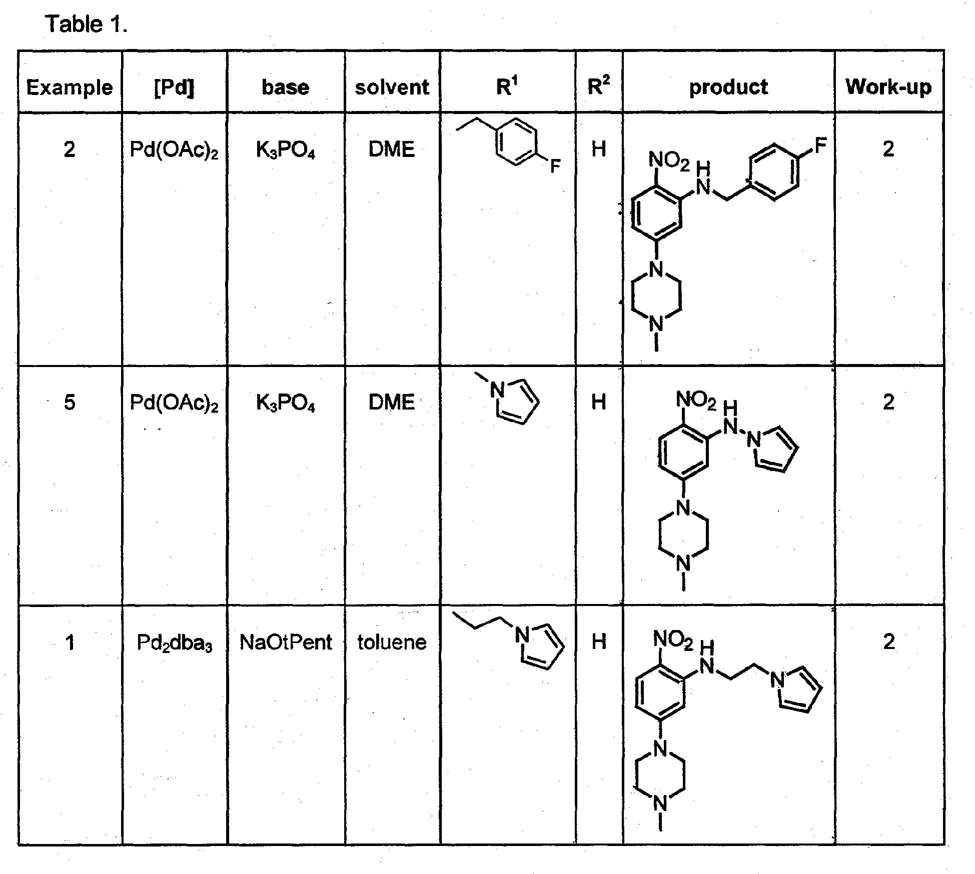

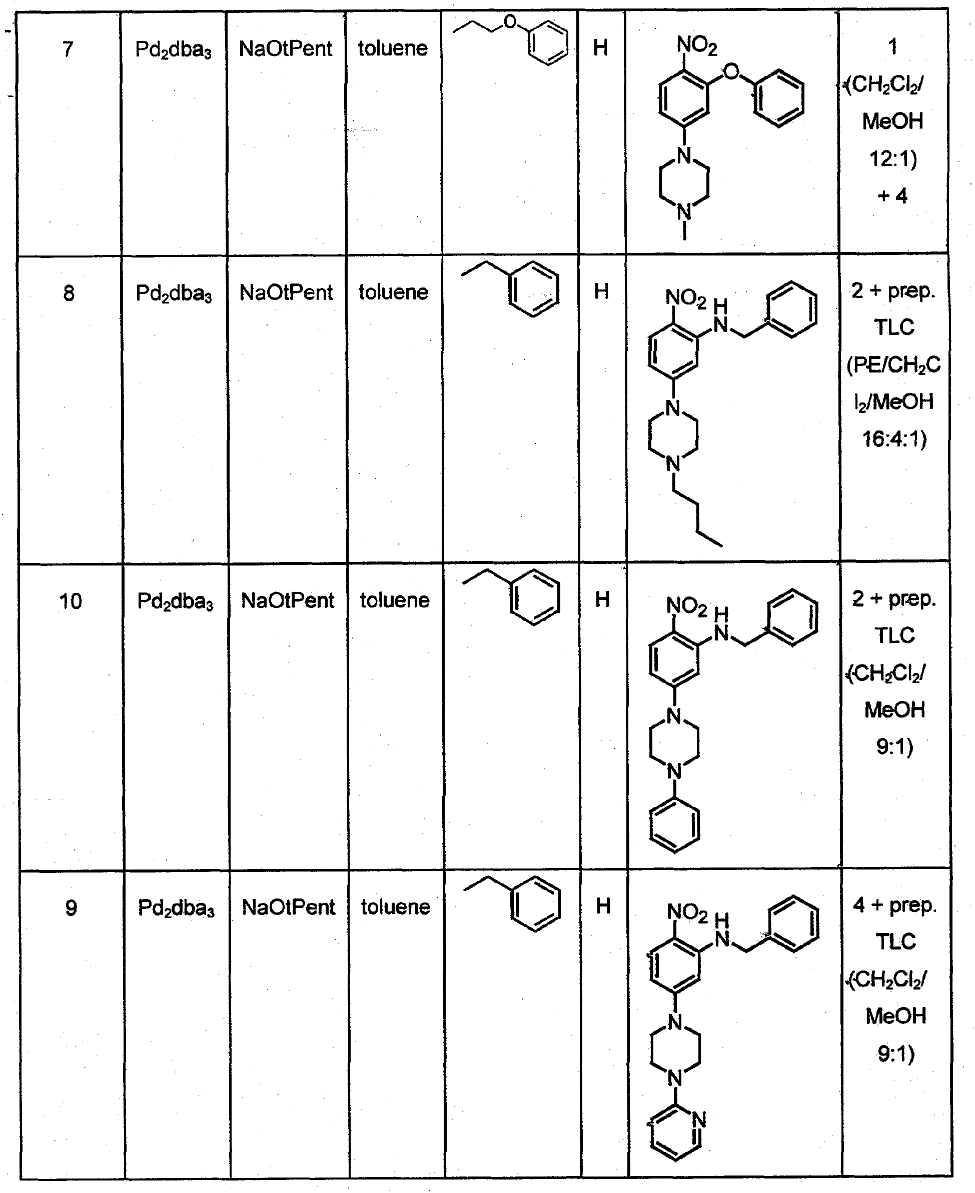

The ¹H-NMR for the example compounds 1, 2, 5, 7, 8, 9 and 10 are as follows:
Compound 1. [5-(4-Methyl-piperazin-1-yl)-2-nitro-phenyl]-(2-pyrrol-1-yl-ethyl)-amine ¹H (300 MHz, CDCl₃): δ 8.48 (*br* s*,* 1 H), 8.06 (d, 1H), 6.71 (dd, 2H), 6.22 (dd, 1H), 6.17 (dd, 2H), 5.69 (d, 1H), 4.20 (dd, 2H), 3.62 (dd, 2H), 3.39 (dd, 4H), 2.54 (dd, 4H), 2.37 (s, 3H)
Compound 2. (4-Fluoro-benzyl)-[5-(4-methyl-piperazin-1-yl)-2-nitro-phenyl]-amine ¹H (300 MHz, CDCl₃): δ 8.72 (*br* s, 1H), 8.08 (d, 1H), 7.33 (t, 2H), 7.05 (t, 2H), 6.22 (dd, 1H), 5.82 (d, 1H), 4.85 (d, 2H), 3.36 (dd, 4H), 2.6 (*br* s, 4H), 2.41 (s, 3H)
Compound 5. [5-(4-Methyl-piperazin-1-yl)-2-nitro-phenyl]-pyrrol-1-yl-amine ¹H (300 MHz, CDCl₃/CD₃OD): δ 8.17 (s, 1H), 8.10 (d, 1H), 6.76 (dd, 2H), 6.46 (dd, 1H), 6.25 (dd, 2H), 5.36 (d, 1H), 3.47 (dd, 4H), 3.10 (dd, 4H), 2.76 (s, 3H)
Compound 7. 1-Methyl-4-(4-nitro-3-phenoxy-phenyl)-piperazine ¹H (300 MHz, CDCl₃): δ 8.06 (d, 1H), 7.35 (t, 2H), 7.14 (t, 1H), 7.00 (*br* d, 2H), 6.60 (dd, 1H), 6.34 (d, 1H), 3.30 (t, 4H), 2.48 (t, 4H), 2.32 (s, 3H)
Compound 8. Benzyl-[5-(4-butyl-piperazin-1-yl)-2-nitro-phenyl]-amine ¹H (300 MHz, d₆-acetone): δ 8.78 (*br* s, 1H), 7.98 (d, 1H), 7.46 (d, 2H), 7.38 (m, 2H), 7.31 (m, 1H), 6.38 (dd, 1H), 6.08 (d, 1H), 4.65 (d, 2H), 3.36 (t, 4H), 2.45 (t, 4H), 2.32 (t, 2H), 1.49 - 1.33 (m, 4H), 0.91 (t, 3H)
Compound 9. Benzyl-[2-nitro-5-(4-pyridin-2-yl-piperazin-1-yl)-phenyl]-amine ¹H (300 MHz, CDCl₃): δ 8.21 (*br* s, 1H), 8.11 (d, 1H), 7.98 (d, 1H), 7.54 - 7.46 (m, 1H), 7.48 (d, 2H), 7.37 - 7.22 (m, 3H), 6.69 - 6.64 (m, 1H), 6.45 (dd, 1H), 6.24 (dd, 1H), 5.85 (d, 1H), 4.53 (d, 2H), 3.77 - 3.63 (m, 6H), 3.46 (m, 2H)
Compound 10. Benzyl-[2-nitro-5-(4-phenyl-2-yl-piperazin-1-yl)-phenyl]-amine ¹H (300 MHz, CDCl₃): δ 8.78 (*br* s, 1H), 8.14 (d, 1H), 7.47 - 7.27 (m, 10H), 6.24 (dd, 1H),5.91 (d, 1H), 4.54 (d, 2H), 3.82 (m, 4H), 3.39 (m, 2H), 3.05 (m, 2H)

### Preparation of example compounds 3, 4, 6 and 21

The starting material 2-bromo-4-chloro-1-nitro-benzene was obtained by oxidation from 2-bromo-4-chloro-aniline according a method described in the reference by A. McKillop et al., Tetrahedron 1987, 43, 1753.

### Step 1.

### Preparation of intermediate compounds 3-I, 4-I and 6-I

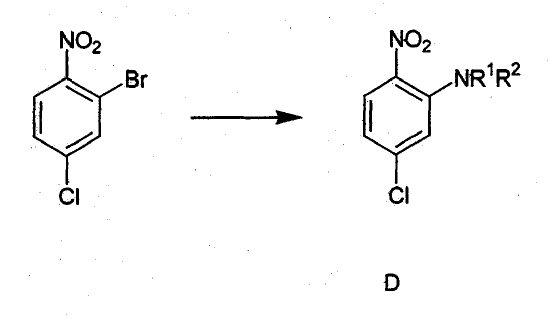

An oven-dried vial was subsequently charged with a palladium source [Pd], xantphos (1.5 eq. to Pd), 1.0 eq. 2-bromo-4-chloro-1-nitro-benzene, 1.1 eq. amide HNR¹R² and 1.4 eq. base. The tube was evacuated, purged with argon and dioxane was added to obtain a concentration of 1.0 M for 2-bromo-4-chloro-1-nitro-benzene. The vial was sealed and heated to 110°C for 21 h. The mixture was worked up differently.

### Work-up 1:

The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA) mixtures.

### Work-up 2:

The reaction mixture was taken up in half-saturated brine and extracted three times with ethyl acetate. The combined organic phases were dried over MgSO₄, concentrated and purified by preparative TLC (1 mm silica gel plate per 0.4 mmol starting aryl halide) using PE/EA mixtures.

### Work-up 3:

The reaction mixture was transferred into a flask using H₂O and MeOH. The total volume was reduced to less than 10 mL and purification was achieved by preparative HPLC. In order to remove any ammonium formate derivative formed during preparative HPLC on RP18, the resulting product was taken up in chloroform, extracted twice with saturated aq. NaHCO₃, the combined aq. phases were re-extracted with chloroform, and the combined organic phases were dried over MgSO₄.

Said process was carried out according to the methode described in the reference of S.L. Buchwald et al. J. Am. Chem. Soc. 2002, 124, 6043-6048. The respective part of the description is hereby incorporated by reference and forms part of the present disclosure.

The different reaction conditions and work-up procedures are listed in the following table 2.

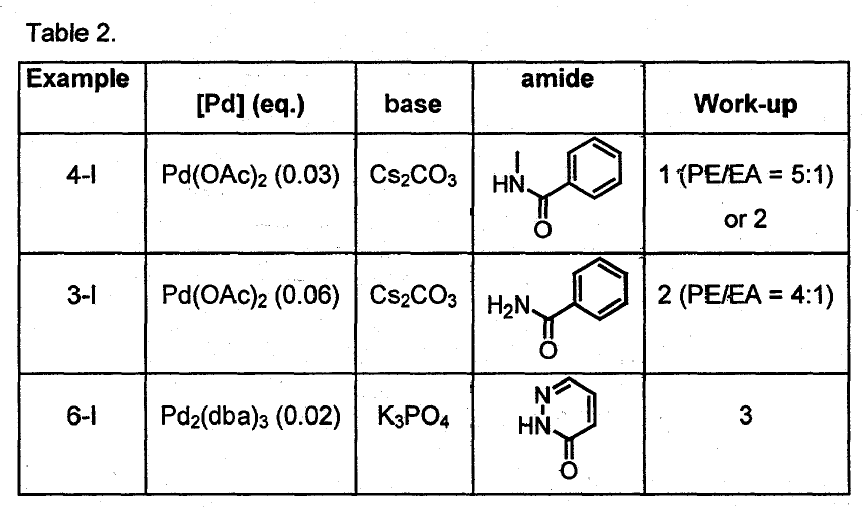

### Preparation of intermediate compound 21-1

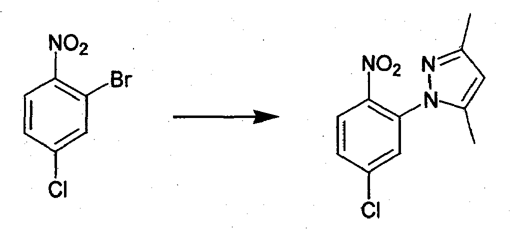

An oven-dried vial was subsequently charged with 0.1 eq. Cul, 1:0 eq. 2-bromo-4-chloro-1-nitro-benzene, 1.2 eq. 3,5-dimethylpyrazole and 2.1 eq. K₃PO₄. The vial was evacuated and purged with argon and 0.3 eq. *trans*-1,2-diaminomethyl-cyclohexane was added. Toluene was added to obtain a concentration of 1.0 M for 2-bromo-4-chloro-1-nitro-benzene. The vial was sealed and heated to 110°C for 21 h.
The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA 5:1 VolumeNolume) mixtures.

Said process was carried out according to the methode described in the reference of S.L. Buchwald et al. J. Am. Chem. Soc. 2002, 124, 11684-11688. The respective part of the description is hereby incorporated by reference and forms part of the present disclosure.

### Preparation of example compounds 4, 5, 6 and 21

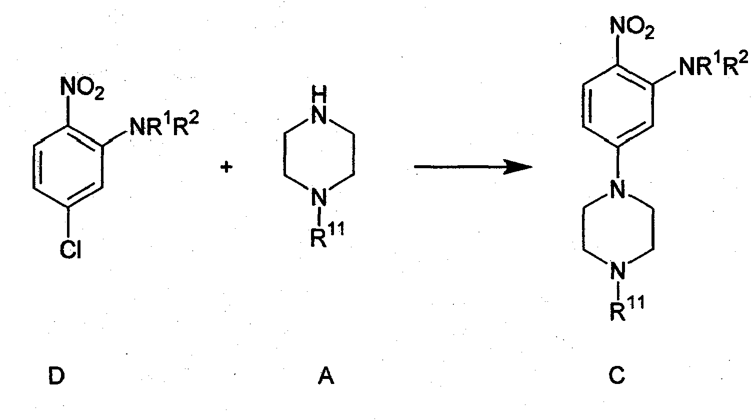

An oven-dried vial was subsequently charged with 0.1 eq. of a palladium source [Pd], 0.2 eq. (biph)P(*t*Bu)₂, 1.0 eq. arylhalide D and 1.4 eq. base. The vial was evacuated and purged with argon, and the respective piperazine A (1.3 eq.) was added. The solvent was added to obtain a concentration of 1.0 M of the arylhalide D. The vial was sealed and heated to either 100°C in case of toluene as the solvent or 110°C in case of DME as the solvent in each case for 22 h. The reaction mixture was transferred into a flask using H₂O and MeOH and the total volume was reduced to less than 10 mL. Purification was achieved by preparative HPLC on RP18. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

Said process was carried out according to the methode described in the reference of S.L. Buchwald et al. J. Am. Chem. Soc. 2002, 124, 6043-6048. The respective part of the description is hereby incorporated by reference and forms part of the present disclosure.

The different reaction conditions are listed in the following table 3.

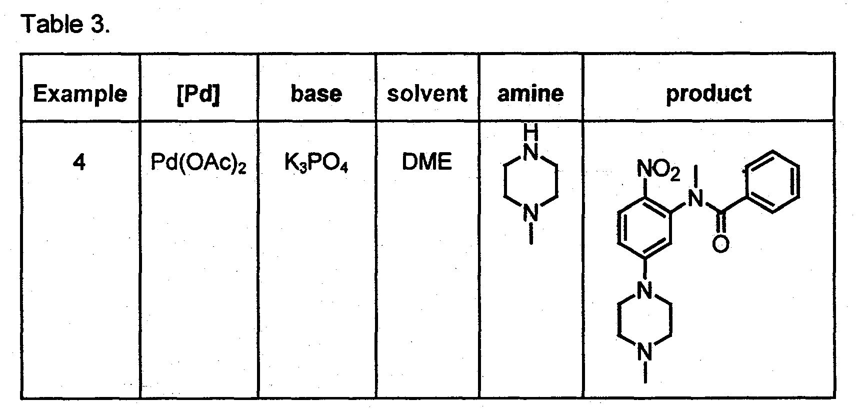

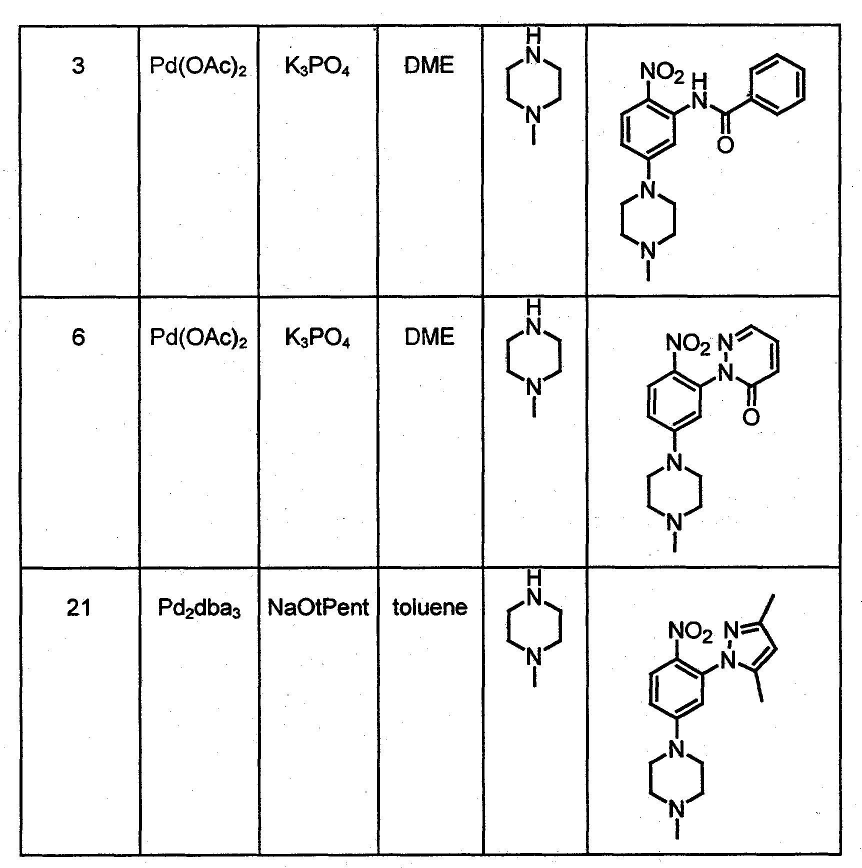

The ¹H-NMR for the example compounds 3, 4 and 6 are as follows:
Compound 3. N-[5-(4-Methyl-piperazin-1-yl)-2-nitro-phenyl]-benzamide ¹H (300 MHz, CDCl₃): δ 12.02 (s, 1H), 8.59 (d, 1H), 8.21 (d, 1H), 8.02 - 7.99 (m, 2H), 7.60 - 7.50 (m, 3H), 6.57 (dd, 1H), 3.57 (dd, 4H), 2.58 (dd, 4H), 2.38 (s, 3H)
Compound 4. N-Methyl-N-[5-(4-methyl-piperazin-1-yl)-2-nitro-phenyl]-benzamide ¹H (300 MHz, CDCl₃): δ 7.94 (*br* s, 1H), 7.28 - 7.18 (m, 5H), 6.63 *(br s,* 1H), 6.45 *(br* s, 1H), 3.38 (br s, 3H), 3.29 (*br* s, 4H), 2.48 (*br* s, 4H), 2.33 (s, 3H)
Compound 6. 2-[5-(4-Methyl-piperazin-1-yl)-2-nitro-phenyl]-2H-pyridazin-3-one ¹H (300 MHz, CDCl₃): δ 8.16 (d, 1H), 7.87 (dd, 1H), 7.28 (dd, 1H), 7.03 (dd, 1H), 6.88 (dd, 1H), 6.79 (d, 1H), 3.45 (t, 4H), 2.54 (t, 4H), 2.35 (s, 3H)
Compound 21. 1-[3-(3,5-Dimethyl-pyrazol-1-yl)-4-nitro-phenyl]-4-methyl-piperazine

The compounds of the examples 11 to 20 and 22 to 60 have been prepared accordingly. Those skilled in the art will realize which starting materials were used to obtain the respective compounds.
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyi-piperazin-1-yl)-2-nitro-phenyl]-amine,
[29] Benzhydryl-[5-(4-methyl-piperazin-1-yl)-2-nitro-phenylj-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine and
[60] 1-[4-(3-Benzylamino-4-nitro-phenyl)-piperazin-1-yl]-ethanone.

### Pharmacological data:

The binding of the nitro-substituted phenyl-piperazine compounds to the 5-HT₆ receptor was determined as described above.

The binding results for some of these compounds are given in the following table :

| **Compound according to example:** | **K**_{**i**} **(nM)** |
|---|---|
| 2 | 172.4 |
| 5 | 480.8 |
| 7 | 26.6 |
| 11 | 61.2 |
| 12 | 6332.2 |
| 13 | 162.5 |
| 15 | 2710.1 |
| 16 | 732.0 |
| 17 | 5002.7 |
| 21 | 249.5 |
| 22 | 27.5 |
| 23 | 118.5 |
| 24 | 1266.7 |
| 26 | 1511.5 |
| 27 | 3778.8 |
| 29 | 4489.0 |
| 30 | 114.1 |
| 31 | 1710.1 |
| 32 | 481.8 |
| 33 | 33.1 |
| 45 | 80.3 |
| 46 | 245.2 |
| 51 | 19.1 |
| 52 | 8.3 |
| 53 | 34.3 |
| 55 | 26.1 |
| 56 | 59.0 |
| 57 | 25.0 |
| 58 | 289.9 |
| 59 | 11.9 |

## Claims

**1.** A nitro-substituted phenyl-piperazine compound of general formula I, 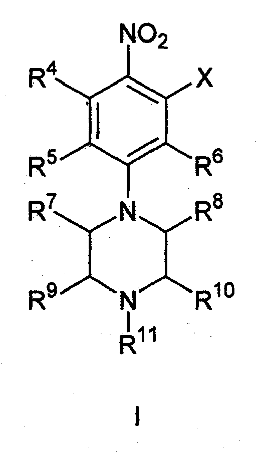 wherein
X represents a -NR¹R² moiety or a -OR³ moiety;
R¹ represents a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -NO₂,-CHO, -CF₂H, -CFH₂, -C(=O)-NH₂, -C(=O)-NH(C₁₋₅-alkyl), -O(=O)-N(C₁₋₅₋alkyl)₂, -S(=O)₂-C₁₋₅-alkyl, -S(=O)₂-phenyl, phenyl, phenoxy and benzyl and which may contain 1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, -NH-(C₁₋₅-alkyl) and -N(C₁₋₅-alkyl)₂;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be substituted with 1, 2, 3, 4 or 5 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl,-O-C(=O)-C₁₋₅-alkyl, F, Cl, Br, I, -CN, -CF₃, -OCF₃, -SCF₃, -OH, -SH,-NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a-(CH₂)-group and may be substituted with 1, 2, 3, 4 or 5 substituent(s) independently selected from the group consisting of C₁₋₅-alkyl, -O-C₁₋₅₋alkyl, -S-C₁₋₅-alkyl, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂; -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2, 3, 4 or 5 substituent(s) independently selected from the group consisting of C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, phenyl, phenoxy and benzyl;
or
a -C(=O)-R¹² moiety;
R² represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, -NH-(C₁₋₅-alkyl) and -N(C₁₋₅-alkyl)₂; or
R¹ and R² together with the bridging nitrogen atom form a saturated or unsaturated, but not aromatic 3- to 6-membered heterocyclic ring which contains 1, 2 or 3 additional heteroatom(s) independently selected from the group consisting of nitrogen and sulfur as ring member(s) and which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl,-O-C(=O)-C₁₋₅-alkyl, F, Cl, Br, I, -CN, -CF₃, -OCF₃, -SCF₃, -OH, -SH,-NH₂, -NH(C₁₋₅-alkyl), -N(C₁₋₅-alkyl)₂, -N02, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl;
R³ represents a 5- to 10-membered aryl or heteroaryl radical, which may be unsubstituted or may be substituted with 1, 2, 3, 4 or 5 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl,-O-C(=O)-C₁₋₅-alkyl, F, Cl, Br, I, -CN, -CF₃, -OCF₃, -SCF₃, -OH, -SH,-NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁴, R⁵ and R⁶ each represent a hydrogen atom;
R⁷ and R⁸, identical or different, each represent a hydrogen atom or a linear or branched, saturated or unsaturated C₁₋₁₀ aliphatic radical which maybe unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and-N(C₁₋₅-alkyl)₂;
R⁹ and R¹⁰, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN,-CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
R¹¹ represents a linear or branched, saturated or unsaturated, unsubstituted C₁₋₁₀ aliphatic radical or a 5- to 10-membered aryl or heteroaryl radical which may be unsubstituted or may be substituted with 1, 2, 3, 4 or 5 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃,-OH, -SH, -NH₂, -NH(C₁₋₅-alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl and whereby the heteroaryl radical contains 1, 2 or 3 heteroatom(s) independently selected from the group consisting of nitrogen, oxygen and sulfur as ring member(s);
and
R¹² represents a 5 to 10 membered aryl radical which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl,-C(=O)-O-C₁₋₅-alkyl, -O-C(=O)-C₁₋₅-alkyl, F, Br, I, -CN, -CF₃,-OCF₃, -SCF₃, -SH, -NH₂, -NH(C₁₋₅-alkyl), -N(C₁₋₅-alkyl)₂, -CHO, -CF₂H,-CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN,-CF₃, -OCF₃, -SCF₃, -OH, -SH, -NH₂, -NH(C₁₋₅-alkyl), -N(C₁₋₅-alkyl)₂,-NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, phenyl, phenoxy and benzyl and which is bonded via a -(CH₂)₁, _{2 or 3}- group;
with the proviso that the following compound
(1) Benzyl-[5-(4-methyl-piperazin-1-yl)-2-nitro-phenyl]-amine is excluded;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

**2.** A compound according to claim 1, **characterized in that** R¹ represents
an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a -(CH₂)_{1, 2 or 3-}group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂₋CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, =SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl; thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃,-N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃₋group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -N02, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or
a -C(=O)-R¹² moiety;
preferably R¹ represents
an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -0-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂,-C(=O)-NH-CH₃, -C(=O)-NH-C₂H₅, -C(=O)-N(CH₃)₂, -C(=O)-N(C₂H₅)₂ and - S(=O)₂-CH₃; or
a -C(=O)-R¹² moiety;
more preferably R¹ represents an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or
a -C(=O)-R¹² moiety.

**3.** A compound according to claim 1 or 2, **characterized in that**
R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical.

**4.** A compound according to one or more of claims 1 to 3, **characterized in that** R¹ and R² together with the bridging nitrogen atom form a moiety selected from the group consisting of 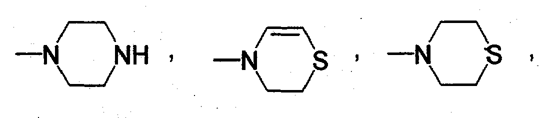 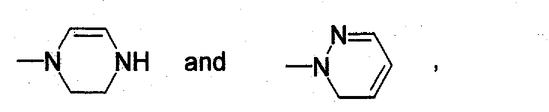 whereby each of these afore mentioned cyclic moieties may be substituted in any position including the -NH-groups with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-O(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl;
preferably R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, - C(=O)-fl-C₂H₅, -C(=O)-CH₃, -C(=O)-C₂H₅, F, CI, Br, I, -CN, -CF₃, -OCF₃, - SCF₃, -OH, -SH, -NH₂, -NH-CH₃, -NH-C₂H₅, -NO₂, -CHO, -CF₂H, -CFH₂, - C(=O)-NH₂, phenyl, phenoxy and benzyl;
more preferably R¹ and R² together with the bridging nitrogen atom form an unsubstituted pyridazin-3-one moiety.

**5.** A compound according to one or more of claims 1 to 4, **characterized in that**
R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1, 2 or 3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -0-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -0-CH₃, -O-C₂H₅, -O-CH₂-CH2-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH3)₂, -C(=O)-N(C₂H₅)₂ and -S(=O)₂-CH₃;
more preferably R³ represents an unsubstituted phenyl radical.

**6.** A compound according to one or more of claims 1 to 5, **characterized in that**
R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom.

**7.** A compound according to one or more of claims 1 to 6, **characterized in that** R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom.

**8.** A compound according to one or more of claims 1 to 7, **characterized in that**
R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹¹ represents an alkyl radical selected from the group consisting of methyl, ethyl, n-propyl and n-butyl; or an unsubstituted phenyl or pyridinyl radical.

**9.** A compound according to one or more of claims 1 to 8, **characterized in that**
R¹² represents a 5 to 10 membered aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -O(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂,-C(=O)-NH-CH₃, -C(=O)-NH-C₂H₅, -C(=O)-N(CH₃)₂, -C(=O)-N(C₂H₅)₂ and - S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benza[b]furanyl, benzo[b]thiophenyl, benzothiadiazolyl and imidazo[2,1-b]thiazolyl, whereby said aryl or heteroaryl radical is bonded via a -(CH₂)_{1, 2 or 3}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -0-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, =C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂,-C(=O)-NH-CH₃, -C(=O)-NH-C₂H₅, -C(=O)-N(OH₃)₂, -C(=O)-N(C₂H₅)₂ and - S(=O)₂-CH₃;
preferably R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -O(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹² represents an unsubstituted phenyl radical.

**10.** A compound according to one or more of claims 1 to 9, **characterized in that**
X represents a -NR¹R² moiety or a -OR³ moiety;
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a - (CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -0-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or a -C(=O)-R¹² moiety;
R² represents a hydrogen atom or an alkyl radical selected from the group consisting of methyl, ethyl, n-propyl, isopropyl, n-butyl, sec-butyl, isobutyl and tert-butyl;
or
R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, - C(=O)-CH₃, -C(=O)-C₂H₅, F, Cl, Br, I, -CN, -CF₃, -OCF₃, -SCF₃, -OH, -SH, - NH₂, -NH-CH₃, -NH-C₂H₅, -NO₂, -CHO, -CF₂H, -CFH₂, -C(=O)-NH₂, phenyl, phenoxy and benzyl;
R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -0-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R⁴, R⁵ and R⁶ each represent a hydrogen atom;
R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and
R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

**11.** A compound according to one or more of claims 1 to 10, **characterized in that**
X represents a -NR¹R² moiety or a -OR³ moiety;
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a - (CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or a -C(=O)-R¹² moiety;
R² represents a hydrogen atom or a methyl radical;
or
R¹ and R² together with the bridging nitrogen atom form an unsubstituted pyridazin-3-one moiety;
R³ represents an unsubstituted phenyl radical;
R⁴, R⁵ and R⁶ each represent a hydrogen atom;
R⁷ and R⁸ each represent a hydrogen atom;
R9 and R¹⁰ each represent a hydrogen atom;
R¹¹ represents an alkyl radical selected from the group consisting of methyl, ethyl, n-propyl and n-butyl; or an unsubstituted phenyl or pyridinyl radical
and
R¹² represents an unsubstituted phenyl radical.

**12.** A compound according to one or more of claims 1 to 11 selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
optionally in form of a physiologically acceptable salt thereof, or a corresponding solvate thereof.

**13.** Process for the preparation of a compound according to one or more of claims 1 to 12, **characterized in that** at least one nitrobenzene compound of general formula II, 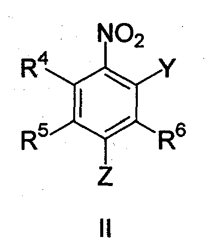 wherein R⁴ to R⁵ have the meaning according to one or more of claims 1 to 12, Y represents a chlorine atom, and Z represents a bromine or iodine atom; is reacted with at least one compound of general formula III, 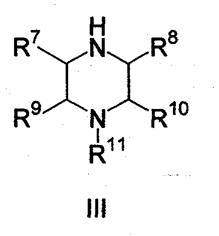 wherein R⁷ to R¹¹ have the meaning according to one or more of claims 1 to 12, in a suitable reaction medium, preferably in the presence of at least one catalyst and/or at least one auxiliary agent and/or at least one base to yield a compound of general formula IV, 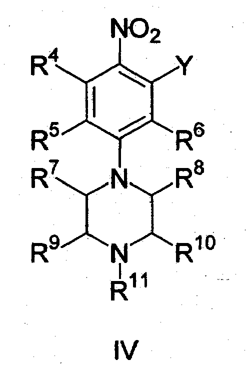 wherein R⁴ to R¹¹ have the meaning according to one or more of claims 1 to 12 and Y represents a chlorine atom; which is optionally purified and/or isolated, and the compound of general formula IV is reacted with at least one compound of general formula V, 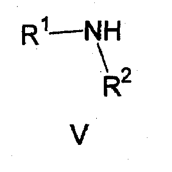 wherein R¹ and R² have the meaning according to one or more of claims 1 to 12, in a suitable reaction medium, preferably in the presence of at least one catalyst and/or at least one auxiliary agent and/or at least one base to yield a compound of general formula VI, 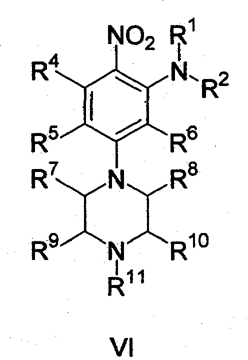 wherein R¹, R² and R⁴ to R¹¹ have the meaning according to one or more of claims 1 to 12, which is optionally purified and/or isolated.

**14.** Process for the preparation of a compound according to one or more of claims 1 to 12, **characterized in that** at least one nitrobenzene compound of general formula VII, 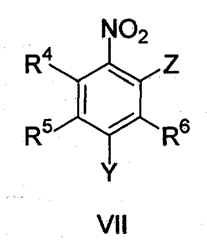 wherein R⁴ to R⁶ have the meaning according to one or more of claims 1 to 12, Z represents a bromine or iodine atom, and Y represents a chlorine atom, is reacted with at least one compound of general formula V, 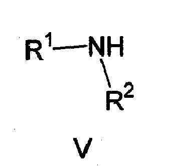 wherein R¹ and R² have the meaning according to one or more of claims 1 to 12, in a suitable reaction medium, preferably in the presence of at least one catalyst and/or at least one auxiliary agent and/or at least one base to yield a compound of general formula VIII, 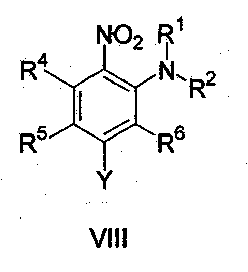 wherein R¹, R² and R⁴ to R⁶ have the meaning according to one or more of claims 1 to 12 and Y represents a chlorine atom; which is optionally purified and/or isolated, and the compound of general formula VIII is reacted with at least one compound of general formula III, 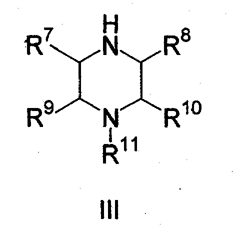 wherein R⁷ to R¹¹ have the meaning according to one or more of claims 1 to 12, in a suitable reaction medium, preferably in the presence of at least one catalyst and/or at least one auxiliary agent and/or at least one base to yield a compound of general formula VI, 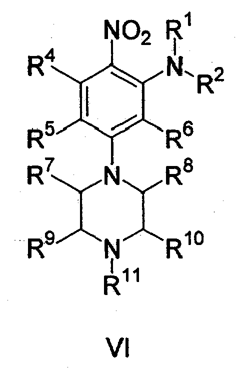 wherein R¹, R² and R⁴ to R¹¹ have the meaning according to one or more of claims 1 to 12, which is optionally purified and/or isolated.

**15.** Process for the preparation of a compound according to one or more of claims 1 to 12, **characterized in that** at least one nitrobenzene compound of general formula II, 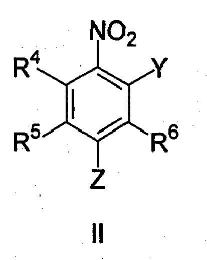 wherein R⁴ to R⁶ have the meaning according to one or more of claims 1 to 12, Y represents a chlorine atom, and Z represents a bromine or iodine atom; is reacted with at least one compound of general formula III, 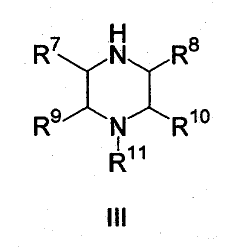 wherein R⁷ to R¹¹ have the meaning according to one or more of claims 1 to 12, in a suitable reaction medium, preferably in the presence of at least one catalyst and/or at least one auxiliary agent and/or at least one base to yield a compound of general formula IV, 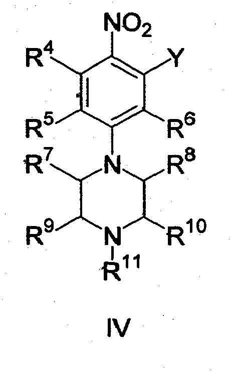 wherein R⁴ to R¹¹ have the meaning according to one or more of claims 1 to 12 and Y represents a chlorine atom; which is optionally purified and/or isolated, and the compound of general formula IV is reacted with at least one compound of general formula IX, 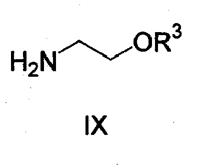 wherein R³ has the meaning according to one or more of claims 1 to 12, in a suitable reaction medium, preferably in the presence of at least one catalyst and/or at least one auxiliary agent and/or at least one base to yield a compound of general formula X, 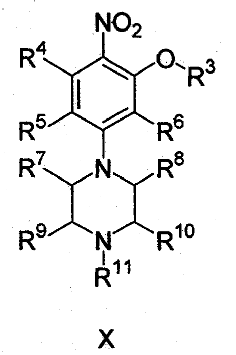 wherein R³ to R¹¹ have the meaning according to one or more of claims 1 to 12, which is optionally purified and/or isolated.

**16.** Medicament comprising at least one nitro-substituted phenyl-piperazine compound of general formula Ia, 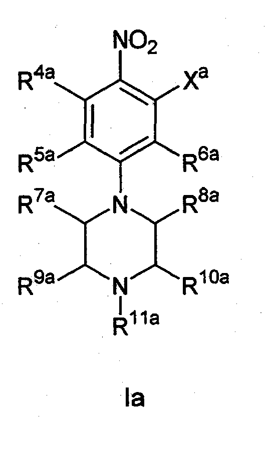 wherein
X^{a} represents a -NR^{1a}R^{2a} moiety or a -OR^{3a} moiety;
R^{1a} represents a linear or branched, saturated or unsaturated C₁₋₁₀ aliphatic radical which is substituted with 1, 2 or 3 substituent(s) independently selected from the group consisting of F, Cl, Br, I, -CN, -CF₃, -OCF₃,-SCF₃, -OH, -SH, -NH₂, -O-C₁₋₅-alkyl, -S-C₁₋₅-alkyl, -NH-(C₁₋₅-alkyl) and-N(C₁₋₅-alkyl)₂;
a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl,-O-C₁₋₅-alkyl, -S-C₁₋₅-alkyl, -C(=O)-OH, -O(=O)-C₁₋₅-alkyl, -C(=O)-O-C₁₋₅₋alkyl, -O-C(=O)-C₁₋₅-alkyl, F, Cl, Br, I, -CN, -CF₃, -OCF₃, -SCF₃, -OH,-SH, -NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅₋alkyl, phenyl, -NH-(C₁₋₅-alkyl) and -N(C₁₋₅-alkyl)₂ and/or which may contain 1, 2 or 3 heteroatom(s) independently selected from the group consisting of nitrogen, oxygen and sulfur as ring member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN,-CF₃, -OCF₃, -SCF₃, -OH, -SH, -NH₂, -NH(C₁₋₅-alkyl), -N(C₁₋₅-alkyl)₂,-NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and whereby said heteroaryl radical contains 1, 2 or 3 heteroatom(s) independently selected from the group consisting of nitrogen, oxygen and sulfur as ring member(s);
a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl,-C(=O)-O-C₁₋₅-alkyl, -O-C(=O)-C₁₋₅-alkyl, F, Cl, Br, I, -CN, -CF₃, -OCF₃,-SCF₃, -OH, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, phenyl, -NH-(C₁₋₅-alkyl) and -N(C₁₋₅₋alkyl)₂ and which may contain 1, 2 or 3 heteroatom(s) independently selected from the group consisting of nitrogen, oxygen and sulfur as chain member(s);
a 5- to 10-membered heteroaryl radical, which may be substituted with 1, 2 or 3 substituent(s) independently selected from the group consisting of C₁₋₅-alkyl, O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN,-CF₃, -OCF₃, -SCF₃, -OH, -SH, -NH₂, -NH(C₁₋₅-alkyl), -N(C₁₋₅-alkyl)₂,-NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, phenyl, -NH-(C₁₋₅-alkyl) and -N(C₁₋₅₋alkyl)₂ and which may contain 1, 2 or 3 heteroatom(s) independently selected from the group consisting of nitrogen, oxygen and sulfur as chain member(s) and whereby the heteroaryl radical contains 1, 2 or 3 heteroatom(s) independently selected from the group consisting of nitrogen, oxygen and sulfur as ring member(s);
or a -C(=O)-R^{12a} moiety;
R^{2a} represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, -NH-(C₁₋₅-alkyl) and -N(C₁₋₅-alkyl)₂;
or
R^{1a} and R^{2a} together with the bridging nitrogen form a saturated, unsaturated or aromatic 3- to 6-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, =OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl and which may contain 1, 2 or 3 additional heteroatom(s) independently selected from the group consisting of nitrogen, oxygen and sulfur as ring member(s) and/or which may be condensed with a mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl,-O-C(=O)-C₁₋₅-alkyl, F, Cl, Br, I, -CN, -CF₃, -OCF₃, -SCF₃, -OH, -SH,-NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
whereby the rings of the ring system are 5- 6- or 7-membered and may contain 1, 2 or 3 heteroatom(s) independently selected from the group consisting of nitrogen, oxygen and sulfur;
R^{3a} represents a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH,-C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group and which may be unsubstituted or substituted with 1, 2 or 3 substituent(s) independently selected from the group consisting of F, Cl, Br, I, -CN, -CF₃, -OCF₃,-SCF₃, -OH, -SH, -NH₂, -O-C₁₋₅-alkyl, -S-C₁₋₅-alkyl, -NH-(C₁₋₅-alkyl) and-N(C₁₋₅-alkyl)₂ and whereby the heteroaryl radical contains 1, 2 or 3 heteroatom(s) independently selected from the group consisting of nitrogen, oxygen and sulfur as ring member(s);
R^{4a}, R^{5a} and R^{6a}, identical or different, each represent a hydrogen atom or a halogen atom;
or
R^{4a} and R^{5a} together with the bridging carbon atoms form an unsubstituted 5- or 6- membered heterocyclic ring which contains 1, 2 or 3 heteroatom(s) independently selected from the group consisting of nitrogen, oxygen and sulfur as ring member(s) and which together with the phenyl ring which it is fused with forms a 9- or 1 0-membered bicyclic aromatic ring system;
R^{7a} and R^{8a}, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN,-CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
R^{9a} and R^{10a}, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN,-CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
R^{11a} represents a hydrogen atom; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH,-NH₂, -O-C₁₋₅-alkyl, -S-C₁₋₅-alkyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN,-CF₃, -OCF₃, -SCF₃, -OH, -SH, -NH₂, -NH(C₁₋₅-alkyl), -N(C₁₋₅-alkyl)₂,-NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched, C₁₋₆ alkylene, C₂₋₆ alkenylene or C₂₋₆ alkinylene group which may be unsubstituted or substituted with 1, 2 or 3 substituent(s) independently selected from the group consisting of F, CI, Br, I, -CN, -CF₃, -OCF₃,-SCF₃, -OH, -SH, -NH₂, -O-C₁₋₅-alkyl, -S-C₁₋₅-alkyl, -NH-(C₁₋₅-alkyl) and-N(C₁₋₅-alkyl)₂ and whereby the heteroaryl radical contains 1, 2 or 3 heteroatom(s) independently selected from the group consisting of nitrogen, oxygen and sulfur as ring member(s); a -C(=O)-R^{13a} moiety or a -S(=O)₂-R^{14a} moiety;
R^{12a} represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅₋alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH,-C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃,-SCF₃, -OH, -SH, -NH₂, -O-C₁₋₅-alkyl, -S-C₁₋₅-alkyl, -NH-(C₁₋₅-alkyl) and-N(C₁₋₅-alkyl)₂ and whereby the heteroaryl radical contains 1, 2 or 3 heteroatom(s) independently selected from the group consisting of nitrogen, oxygen and sulfur as ring member(s);
and
R^{13a} and R^{14a}, identical or different, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃,-SCF₃, -OH, -SH, -NH₂, -O-C₁₋₅-alkyl, -S-C₁₋₅-alkyl, -NH-(C₁₋₅-alkyl) and-N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH,-C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN,-CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member{s).
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

**17.** Medicament according to claim 16, **characterized in that**
R^{1a} represents an alkyl radical selected from the group consisting of methyl, ethyl, n-propyl, isopropyl, n-butyl, sec-butyl, isobutyl and tert-butyl which is substituted with 1, 2 or 3 substituent(s) independently selected from the group consisting of F, Cl, Br, I, -CN, -CF₃, -OCF₃, -SCF₃, -OH, -SH and -NH₂;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, phenyl, phenoxy and benzyl;
an aryl radical selected from the group consisting of phenyl and naphthyl which may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl,-S(=O)₂-phenyl, phenyl, phenoxy and benzyl and said aryl radical is bonded via a linear or branched C₁₋₆ alkylene group which may contain 1, 2 or 3 oxygen atom(s) as chain member(s) and which may be substituted with 1 phenyl radical;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s);
or a -C(=O)-R^{12a} moiety;
preferably R^{1a} represents an alkyl radical selected from the group consisting of methyl, ethyl, n-propyl and isopropyl, whereby said alkyl radical is substituted with 1,2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl; imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-O-CH₃, -C(=O)-O-C₂H₅, -C(=O)-O-CH₂-CH₂₋CH3, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-O(CH₃)₃, F, Cl, Br, I, -CN, - CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH₂-CH₂-, -CH₂-CH₂-CH₂₋and -CH₂-CH₂-O-;
or a -C(=O)-R^{12a} moiety;
more preferably R^{1a} represents an alkyl radical selected from the group consisting of -CH₂-CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂₋CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety.

**18.** Medicament according to claim 16 or 17, **characterized in that**
R^{2a} represents a hydrogen atom or a linear or branched, unsubstituted C₁₋₁₀ alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical.

**19.** Medicament according to one or more of claims 16 to 18, **characterized in that**
R^{1a} and R^{2a} together with the bridging nitrogen atom form a moiety selected from the group consisting of 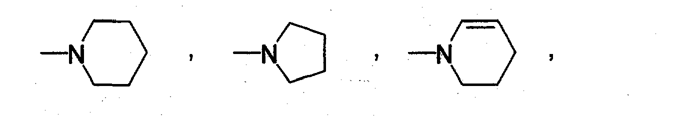 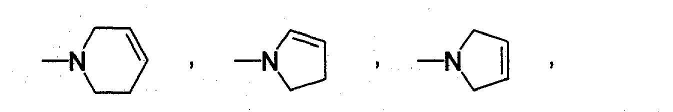 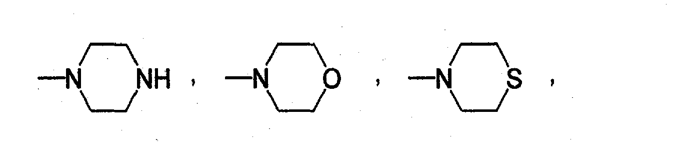 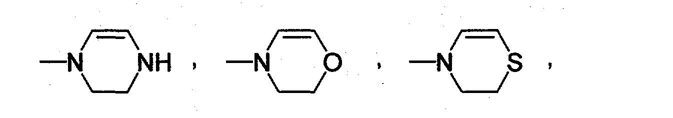 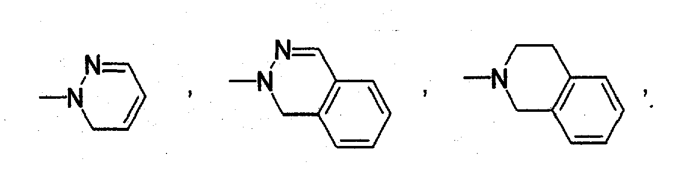 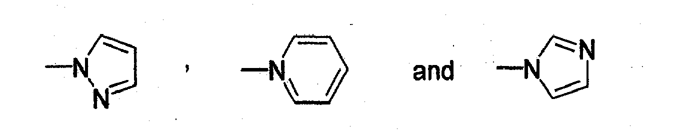 whereby each of these afore mentioned cyclic moieties may be substituted in any position including the -NH-groups with 1, 2 or 3 substituent(s) independently selected from the group consisting of C₁₋₅-alkyl, -O-C₁₋₅-alkyl, - S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and NO₂;
preferably R^{1a} and R^{2a} together with the bridging nitrogen atom form a moiety selected from the group consisting of 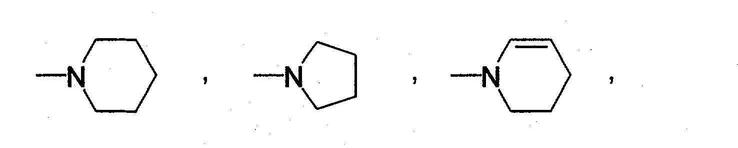 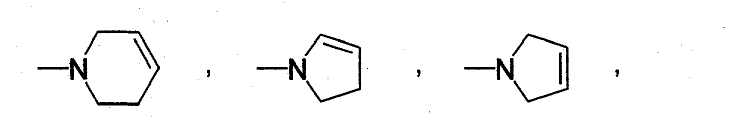 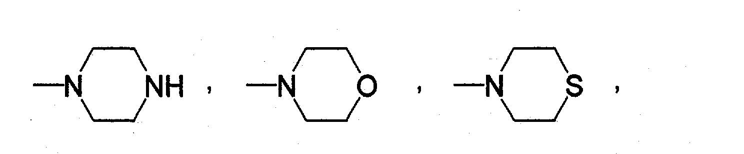 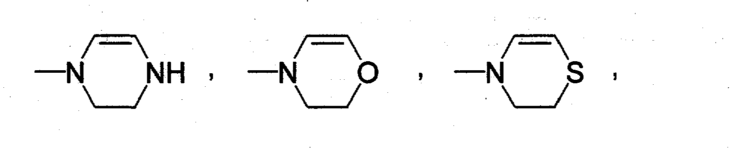 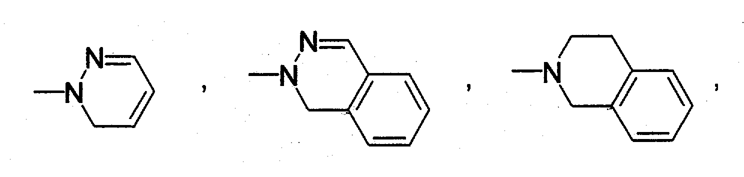 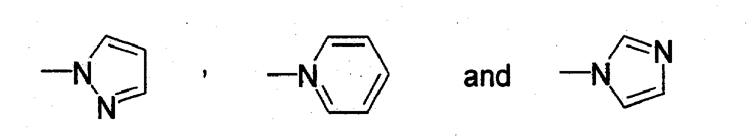 whereby each of these afore mentioned cyclic moieties maybe substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C-(=O)-NH₂, - C(=O)-NH-CH₃, -C(=O)-NH-C₂H₅, -C(=O)-N(CH₃)₂, -C(=O)-N(C₂H₅)₂, -S(=O)₂₋CH₃, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and NO₂;
more preferably R^{1a} and R^{2a} together with the bridging nitrogen atom form a moiety selected from the group consisting of 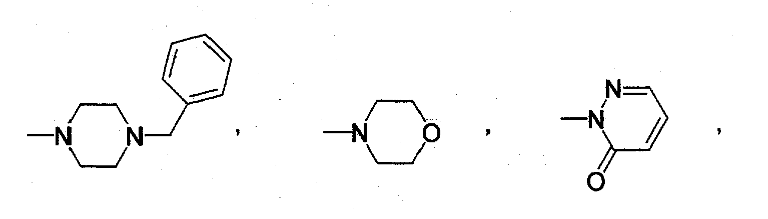 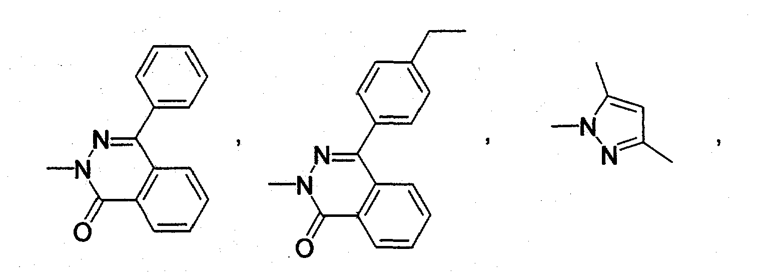 and

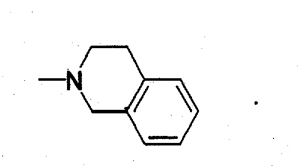

**20.** Medicament according to one or more of claims 16 to 19, **characterized in that**
R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopmpyl, n-butyl, tert-butyl, sec-butyl, isobutyl, n-pentyl, -O-CH₃, -O-C₂H₅, -O-CH₂-CH₂-CH₃, -O-CNCH₃)₂, -0-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂₋CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R^{3a} represents a phenyl radical which may be substituted with 1, 2 or 3 substituent(s) independently selected from the group consisting of methyl, ethyl, n-propyl, isopropyl, n-butyl, tert-butyl, -O-CH₃, -O-C₂H₅, -O-CH₂-CH₂₋CH3, - -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, - CF₂H, -CFH₂ and -S(=O)₂-CH₃;
more preferably R^{3a} represents an unsubstituted phenyl radical.

**21.** Medicament according to one or more of claims 16 to 20, **characterized in that**
R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom.

**22.** Medicament according to one or more of claims 16 to 21, **characterized in that**
R^{4a} and R^{5a} together with the bridging carbon atoms form a moiety selected from the group consisting of 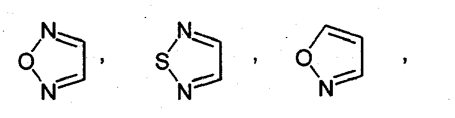 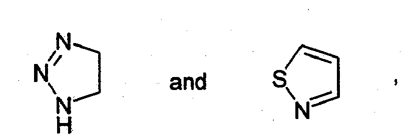 which together with the phenyl ring which it is fused with forms a substituted bicyclic aromatic ring system selected from the group consisting of 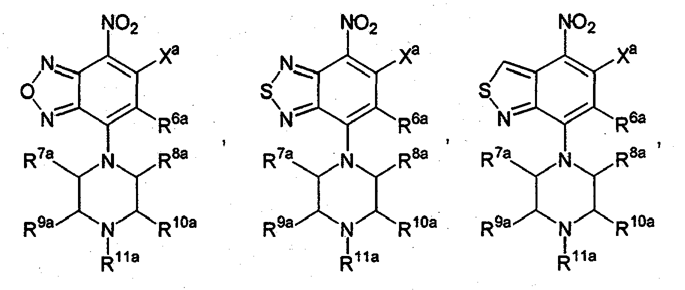 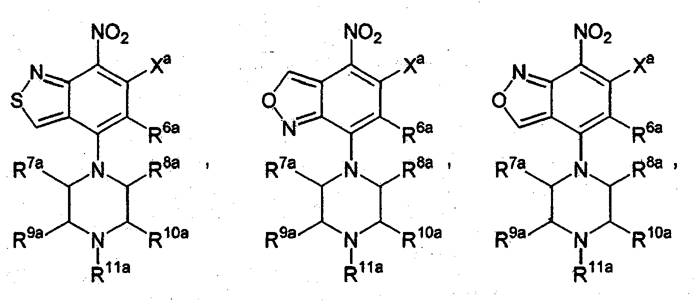 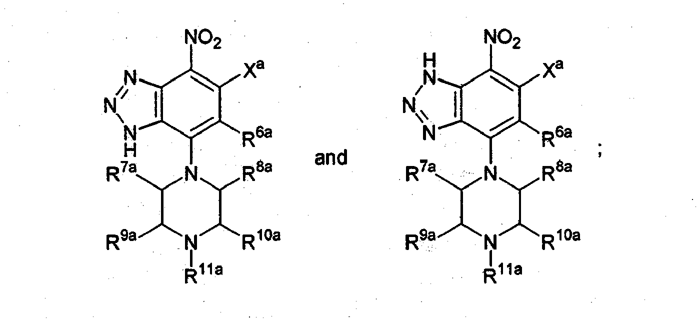 preferably R^{4a} and R^{5a} together with the bridging carbon atoms form the following moiety, 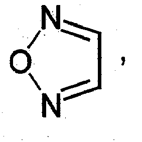 which together with the phenyl ring which it is fused with forms the following substituted bicyclic aromatic ring system

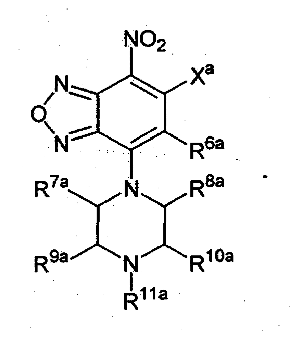

**23.** Medicament according to one or more of claims 16 to 22, **characterized in that**
R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{7a} and R^{8a} each represent a hydrogen atom.

**24.** Medicament according to one or more of claims 16 to 23, **characterized in that**
R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical.

**25.** Medicament according to one or more of claims 16 to 24, **characterized in that**
R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -O(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
a -C(=O)-R^{13a} moiety or a -S(=O)₂-R^{14a} moiety;
preferably R^{11a} represents a hydrogen atom;
an alkyl radical selected from the group consisting of methyl, ethyl, n-propyl, isopropyl, n-butyl, sec-butyl, isobutyl andtert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group;
a -C(=O)-R^{13a} moiety or a -S(=O)₂-R^{14a} moiety;
more preferably R^{11a} represents a hydrogen atom;
an alkyl radical selected from the group consisting of methyl, n-butyl and - CH₂-CH₂-OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety.

**26.** Medicament according to one or more of claims 16 to 25, **characterized in that**
R^{12a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl; isothiazolyl; imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C-(=O)-C₁₋₅₋alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
preferably R^{12a} represents an alkyl radical selected from the group consisting of methyl, ethyl, n-propyl and isopropyl,
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -G(=O)-NH-C₂H₅, -C(=O)-N(CH₃)₂, -C(=O)-N(C₂H₅)₂ and -S(=O)₂-CH₃ and/or may be bonded via a -(CH₂)- group;
more preferably R^{12a} represents a phenyl or a thiophenyl radical whereby said phenyl or thiophenyl radical may be substituted with 1, 2 or 3 substituent(s) selected from the group consisting of methyl and chlorine.

**27.** Medicament according to one or more of claims 16 to 26, **characterized in that**
R^{13a} represents an alkyl radical selected from the group-consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -0-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅; -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group;
preferably R^{13a} represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, and thienyl (thiophenyl), whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group;
more preferably R^{13a} represents a methyl radical or a phenyl or a thiophenyl radical whereby said phenyl or thiophenyl radical may be substituted with 1, 2 or 3 substituent(s) selected from the group consisting of methyl and chlorine.

**29.** Medicament according to one or more of claims 16 to 28, **characterized in that**
R^{14a} represents an alkyl radical selected from the group-consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C-(=O)-C₁₋₅₋alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
preferably R^{14a} represents an alkyl radical selected from the group consisting of methyl, ethyl, n-propyl and isopropyl,
or a phenyl radical, which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -0-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂₋CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R^{14a} represents a methyl radical or a phenyl radical which may be substituted with 1, 2 or 3 methyl radical(s).

**30.** Medicament according to one or more of claims 16 to 29, **characterized in that**
X^{a} represents a -NR^{1a}R^{2a} moiety or a -OR^{3a} moiety;
R^{1a} represents an alkyl radical selected from the group consisting of -CH₂₋CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂₋CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
R^{2a} represents a hydrogen atom or a methyl radical;
or
R^{1a} and R^{2a} together with the bridging nitrogen atom form a moiety selected from the group consisting of 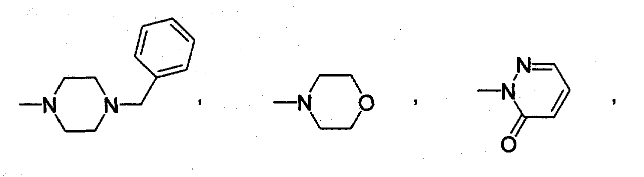 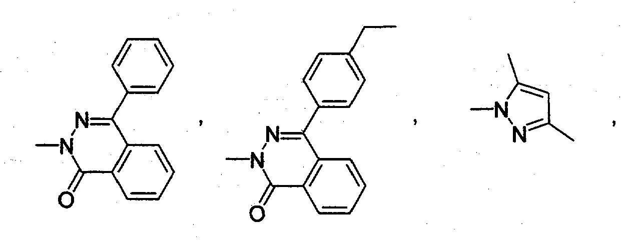 and 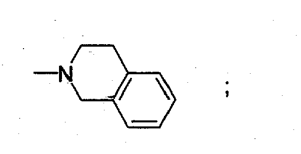 R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 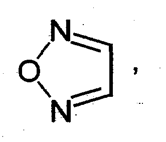 which together with the phenyl ring which it is fused with forms the following substituted bicyclic aromatic ring system 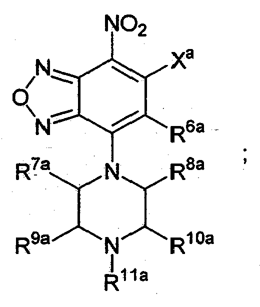 R^{7a} and R^{8a} each represent a hydrogen atom;
R^{9a} and R^{10a}, identical or different, each represent a hydrogen atom or a methyl radical;
R^{11a} represents a hydrogen atom;
an alkyl radical selected from the group consisting of methyl, n-butyl and - CH₂-CH₂-OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety;
R^{12a} represents a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine
and
R^{14a} represents a methyl radical or a phenyl radical which may be substituted with 1, 2 or 3 methyl radical(s);
optionally in form a physiologically acceptable salt thereof, or a corresponding solvate thereof.

**31.** Medicament according to one or more of claims 16 to 30 comprising at least one compound selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1 -yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-pheny]-(2-m-tolyloxy-ethyl)-amine,
[48] [2-(4-Chloro-phenoxy)-ethyl]-[5-(4-methyl-piperazin-1-y))-2-nitrophenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-{5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1 -yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine and
[60] 1-[4-(3-Benzylamino-4-nitro-phenyl)-piperazin-1-yl]-ethanone,
optionally in form of a physiologically acceptable salt thereof, or a corresponding solvate thereof.

**32.** Medicament according to one or more of claims 16 to 31 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

**33.** Use of at least one compound of general formula la according to one or more of claims 16 to 31 for the manufacture of medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

**34.** Use of at least one compound of general formula la according to one or more of claims 16 to 31 for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder).

**35.** Use of at least one compound of general formula la according to one or more of claims 16 to 31 for the manufacture of a medicament for the improvement of cognition (cognitive enhancement) and/or for the improvement of cognitive memory (cognitive memory enhancement).

**36.** Use of at least one compound of general formula Ia according to one or more of claims 16 to 31 for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.
